# FASCICULE DE BREVET EUROPEEN

(11) **EP 1 312 616 B1**
(45) Date de publication et mention de la délivrance du brevet: **23.01.2008**
(21) Numéro de dépôt: 02292601.8
(22) Date de dépôt: 21.10.2002
(51) Int. Cl.: C08B 37/00, C08B 31/04, C08B 35/02, C08B 3/14, A61K 8/00, A61Q 5/00

(54) **Préparation de composés de type bétainates de polysaccharides, composés obtenus, leur utilisation et les compositions les comprenant**
Herstellung von Polysaccharidbetainatverbindungen, hergestellte Verbindungen, deren Verwendung und diese enthaltenden Zusammensetzungen
Preparation of compounds of the kind polysaccharide betainates, obtained compounds, their use and the compositions containing them

(30) Priorité: 15.11.2001 FR 0114789
(43) Date de publication de la demande: 21.05.2003
(73) Titulaire: L'ORÉAL, 75008 Paris (FR)
(72) Inventeur: Dubief, Claude, 78150 Le Chesnay (FR); Rinaudo, Marguerite, 38000 Grenoble (FR); Auzely-Velty, Rachel, 38450 Saint-Georges de Commiers (FR)
(74) Mandataire: Dodin, Catherine

(56) Documents cités:
- WO-A-00/15669
- WO-A-01/62805
- FR-A- 2 370 058
- DATABASE WPI Week 200168 Derwent Publications Ltd., London, GB; AN 2001-602245 XP002207030 & WO 01 46264 A (AJINOMOTO CO), 28 juin 2001 (2001-06-28) & WO 01 46264 A (AJINOMOTO) 28 juin 2001 (2001-06-28)
- PATENT ABSTRACTS OF JAPAN vol. 017, no. 142 (C-1038), 23 mars 1993 (1993-03-23) & JP 04 312596 A (ISHIHARA SANGYO KAISHA LTD), 4 novembre 1992 (1992-11-04)
- DATABASE WPI Week 198206 Derwent Publications Ltd., London, GB; AN 1982-11563E XP002207031 "Polyfunctional physiologically active soluble bio-polymer - by reacting betaine with pectic acid di:aldehyde in ethanol and treating prod. with acetone" & SU 825 542 A (AS KIRG ORG CHEM), 5 mai 1981 (1981-05-05)
- PATENT ABSTRACTS OF JAPAN vol. 1996, no. 08, 30 août 1996 (1996-08-30) & JP 08 092039 A (KANEBO LTD), 9 avril 1996 (1996-04-09)
- PATENT ABSTRACTS OF JAPAN vol. 2000, no. 04, 31 août 2000 (2000-08-31) & JP 2000 001504 A (LIGNYTE CO LTD), 7 janvier 2000 (2000-01-07)

## Description

La présente invention concerne la préparation de composés de la famille des bétaïnates de polysaccharides, et leur utilisation en cosmétique ou en dermatologie. L'invention concerne également les compositions, notamment cosmétiques ou dermatologiques, comprenant ces composés.

Les bétaïnates d'amidon et leurs dérivés sont, pour certains, des composés connus dans la littérature, notamment dans le domaine de la fabrication du papier.

Le document WO01/62805 décrit des polymères hydroxylés sur lesquels sont greffés des ligands cationiques par l'intermédiaire d'une liaison ester, ainsi que des matrices polymériques cationiques obtenues à partir desdits polymères, susceptibles d'être employés comme vecteurs de molécules à activité biologique.

Il est également connu, notamment par la demande de brevet WO 00/15669 et l'article "Preparation of starch betainate : a novel cationic starch derivative" dans Carbohydrate Polymers 41 (2000) 277-283, de synthétiser des bétaïnates d'amidon de pomme de terre, par estérification des groupes hydroxyles de l'amidon avec un chlorure de bétaïnyle. L'estérification est effectuée dans la pyridine et le 1,4-dioxane, à une température d'environ 110°C.

Toutefois, cette synthèse est délicate à mettre en oeuvre, notamment du fait des conditions opératoires et du caractère peu stable des dérivés de bétaïne mis en oeuvre, ce qui altère le rendement de la réaction.

Après de nombreuses recherches, la demanderesse a mis au point deux nouveaux procédés de synthèse de bétaïnates d'amidon, qui permettent une obtention beaucoup plus aisée, avec un rendement amélioré, de ces composés.

De plus, ces procédés permettent également la préparation de nouveaux composés de la famille des bétaïnates de polysaccharides, composés dont les propriétés sont susceptibles d'être exploitées dans les domaines cosmétique ou dermatologique.

Ainsi, l'invention a pour objet un procédé de préparation de composés de formule (I) telle que définie ci-après, dans lequel on fait réagir un polysaccharide avec un acide N,N-dialkylaminocarboxylique de formule (II) telle que définie ci-après, en présence d'au moins un activateur de réaction, puis on quaternise l'ester formé à l'aide d'un agent de quaternisation.

Un autre objet de l'invention est un procédé de préparation de composés de formule (I) telle que définie ci-après, dans lequel on fait réagir un polysaccharide avec un sel d'acide

N,N,N-trialkylammonio-carboxylique de formule (III) telle que définie ci-après, caractérisé par le fait que la réaction est effectuée dans un solvant polaire et aprotique et qu'elle a lieu en présence d'au moins un activateur de réaction.

Un autre objet de l'invention est une composition cosmétique capillaire comprenant dans un milieu cosmétiquement ou dermatologiquement acceptable, au moins un composé de formule (I) telle que définie ci-après, à une concentration comprise entre 0,01 et 20% en poids par rapport au poids total de la composition.

Un autre objet de l'invention est un procédé de traitement cosmétique d'un support kératinique choisi parmi les cheveux, caractérisé par le fait qu'on applique sur ledit support kératinique une composition telle que définie ci-dessus.

Les composés selon la présente invention sont des composés de la famille des bétaïnates de polysaccharides, répondant à la formule (I) : 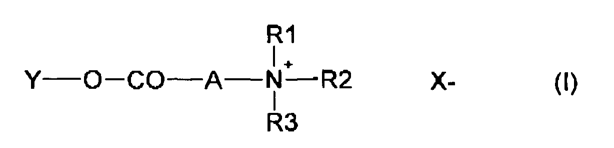 dans laquelle :
- R₁, R₂ et R₃ désignent, indépendamment l'un de l'autre, un radical hydrocarboné saturé ou insaturé, linéaire ou ramifié, en C₁-C₃₀. éventuellement interrompu par un ou plusieurs hétéroatomes choisis parmi N, O et S et/ou éventuellement substitué par un ou plusieurs radicaux choisis parmi -OH, halogène (notamment chlore, brome, iode) et/ou aryle en C₆-C₂₂,
- A désigne un radical hydrocarboné divalent, saturé ou insaturé, linéaire ou ramifié en C₁-C₂₂, éventuellement interrompu par un ou plusieurs hétéroatomes choisis parmi N, O et S, et/ou éventuellement substitué par un ou plusieurs radicaux hydroxyle,
- X⁻ désigne un anion issu d'un acide minéral ou organique, et
- Y désigne un reste polysaccharide,
à l'exclusion des composés dans lesquels Y représente une structure polymère de l'amidon, A représente -CH₂- et R1=R2=R3= -CH₃.

De préférence, R1, R2 et R3 désignent, indépendamment l'un de l'autre, un radical hydrocarboné saturé, linéaire ou ramifié, en C₁-C₁₄, notamment en C₂-C₁₂, et préférentiellement un radical méthyle.

Dans un mode préféré d'exécution, R1, R2 et R3 sont identiques et peuvent notamment être un radical hydrocarboné saturé, linéaire ou ramifié, en C₁-C₁₄. notamment un radical méthyle.

De préférence, A désigne un radical hydrocarboné divalent saturé, linéaire ou ramifié en C₁-C₄, de préférence méthylène, éthylène, propylène, butylène, et préférentiellement méthylène.

Parmi les anions issus d'un acide organique, on peut citer les citrates, les lactates et les tartrates. Parmi les anions issus d'un acide minéral, on peut citer les chlorures, les bromures, les iodures et les sulfates.

De préférence, ces anions sont des chlorures.

Les polysaccharides peuvent être des polysaccharides naturels ou modifiés.

Parmi les polysaccharides naturels, on peut citer les amidons et notamment amidons issus de sources végétales telles que le maïs, la pomme de terre, l'avoine, le riz, le tapioca, le sorgho, l'orge ou le blé; les polysaccharides issus des gommes de graines tells que les gommes de guar, tara, caroube, psyllium, lin, okra, tamarind, coing; les polysaccharides issus des gommes microbiennes telles que les gommes de curdlane, pullulane, dextrane, grifolane, schizophyllane, spirulinane, krestin, xanthane, scleroglucane, gellane, succinoglycane; les polyfructoses tels que l'inuline ou la levane; les extraits d'algues (agar, alginate, caragghénanes, fucoidane, furcellerane, laminarane); les extraits d'exudats de plantes (gomme arabique, ghatti, karaya, adraganthe); les celluloses; les chitines.

On entend par polysaccharides modifiés des polymères obtenus par hydrolyse, estérification, éthérification, amidation, oxydation, réduction, modification des groupements hydroxyle, incorporation covalente de restes organométalliques, greffage, N-acylation, N-alkylation, désamination, ou toute autre modification des fonctions azotées de polysaccharides contenant lesdites fonctions et/ou halogénation selon les procédés décrits dans la littérature.

Parmi ces procédés, on peut citer :
- l'hydrolyse partielle des chaînes polysaccharidiques à l'aide de traitements enzymatiques, thermiques, acides, oxydants;
- l'hydrolyse partielle ou totale de fonctions esters naturellement présentes;
- l'estérification et/ou l'amidation partielle ou totale des fonctions carboxyliques naturellement présentes;
- la carboxylation à l'aide par exemple de monochloroacétate;
- l'oxydation de fonctions hydroxyles du polysaccharide;
- la polyoxyalkylénation;
- la modification hydrophobe d'un polysaccharide par exemple en faisant réagir un agent alkylant en C₁-C₂₂ comme le chlorure de méthyle ou le chlorure de nonyle sur l'alcali cellulose ou sur l'hydroxyéthylcellulose;
- la réticulation de chaînes polysaccharidiques; et/ou
- le greffage par exemple conduisant à l'introduction dans la molécule de motifs tels que polydiméthylsiloxane, acide polyacrylique ou polyméthacrylique, polyacrylamide, polyméthacrylamide, polyacrylonitrile, polystyrène sulfonate de sodium, polyvinylpyrrolidone, polyhydroxy alkyl (méth)acrylate, sel de sodium de l'acide poly(2-acrylamide-2-methyl propane sulfonique).

Parmi les polysaccharides modifiés, on peut citer les hydroxyalkylcelluloses et notamment les hydroxyéthylcelluloses, les hydroxyalkylguars tels que l'hydroxypropylguar, les carboxyalkylcelluloses, les carboxyalkylamidons, les chitosanes modifiés ou non, les maltodextrines et les cyclodextrines.

Les composés selon l'invention peuvent être préparés notamment selon l'un ou l'autre des procédés suivants, objets de la présente invention.

Selon un premier procédé de préparation,
- on fait réagir un polysaccharide avec un acide N,N-dialkylaminocarboxylique de formule (II) : 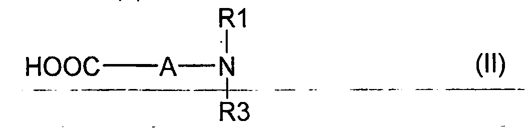 en présence d'au moins un activateur de réaction,
- puis on quaternise l'ester formé à l'aide d'un agent de quaternisation, de manière à fixer le radical R2 sur l'atome d'azote.

Selon ce procédé, on forme dans une première étape un (N,N-dialkyl amino)ester de polysaccharide, qui conduit, dans la seconde étape au sel de N,N,N-trialkylaminoester de polysaccharide.

De préférence, la réaction entre le polysaccharide et l'acide est effectuée dans un solvant adéquat.

De préférence, on dissout ou on disperse le polysaccharide et l'acide N,N-dialkylaminocarboxylique dans ledit solvant adéquat, avant ladite réaction.

De préférence, on dissout ou on disperse le polysaccharide et l'acide dans ledit solvant, avant l'addition de l'activateur de réaction.

Ledit solvant est de préférence polaire et aprotique, et peut notamment être choisi parmi le diméthyl sulfoxyde (DMSO) et/ou le diméthylformamide.

De préférence, l'activateur der réaction est choisi parmi les carbodiimides tels que le N,N'-diisopropylcarbodiimide; l'hydroxybenzotriazole, le 4-diméthylamino-pyridine et leurs mélanges.

De préférence, la réaction est effectuée à température ambiante.

L'agent de quaternisation employé peut être tout agent de quaternisation connu de l'homme du métier. En particulier, cet agent peut être un halogénure tel qu'un chlorure, un iodure ou un bromure, et notamment un halogénure d'alkyle en C1-C30, ou bien peut être un sulfate, notamment un sulfate d'alkyle en C1-C30. De préférence, l'agent de quaternisation est choisi parmi l'iodure de méthyle (Mel) ou le sulfate de méthyle (Me₂ SO₄).

De préférence, la quaternisation est effectuée à une température comprise entre 20 et 70°C, préférentiellement comprise entre 22 et 27°C.

La quaternisation peut être effectuée sur un composé isolé, et/ou dans un autre milieu solvant, différent du solvant de réaction.

Selon un second procédé de préparation, on fait réagir un polysaccharide avec un sel d'acide N,N,N-trialkylammonio-carboxylique de formule (III) 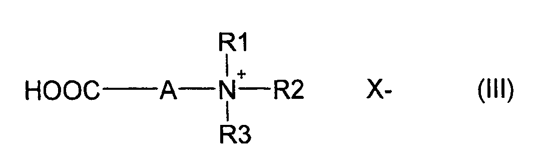 caractérisé par le fait que :
- la réaction est effectuée dans un solvant polaire et aprotique, et
- la réaction a lieu en présence d'au moins un activateur de réaction.

De préférence, on dissout ou l'on disperse le polysaccharide et le sel d'acide dans ledit solvant polaire et aprotique, avant la réaction.

De préférence, le solvant polaire et aprotique est choisi parmi le diméthyl sulfoxyde (DMSO) et/ou le diméthylformamide.

De préférence, on dissout ou on disperse le polysaccharide et le sel d'acide dans le solvant, avant l'addition de l'activateur de réaction.

De préférence, l'activateur de réaction est choisi parmi les carbodiimides tels que le N,N'-diisopropylcarbodiimide; l'hydroxybenzotriazole, le 4-diméthylaminopyridine et leurs mélanges.

De préférence, la réaction est effectuée à température ambiante.

De préférence, la réaction est conduite pendant environ 3 à 24 heures.

Les polysaccharides susceptibles d'être employés dans l'un ou l'autre de ces deux procédés sont similaires à ceux listés ci-dessus.

Parmi les acides N,N-dialkylaminocarboxyliques susceptibles d'être employés dans le premier procédé, on peut citer la N,N-diméthyl glycine (ou acide N,N-diméthyl-aminoacétique), l'acide N,N-diméthyl-β-aminopropanoïque, l'acide N,N-diméthyl-γ-aminobutanoïque, l'acide N,N-diméthyl-5-aminopentanoïque, l'acide N,N-diméthyl benzylamine carboxylique.

De préférence, l'acide N,N-dialkylaminocarboxylique est la N,N-diméthyl glycine.

Parmi les sels d'acides N,N,N-trialkylammoniocarboxyliques susceptibles d'être employés dans le second procédé, on peut citer les sels de l'acide N,N,N-triméthyl-ammonioacétique (ou N,N,N-triméthyl glycine), de l'acide N,N,N-triméthyl-β-ammoniopropanoïque, de l'acide N,N,N-triméthyl-γ-ammoniobutanoïque, de l'acide N,N,N-triméthyl-5-ammoniopentanoïque, de l'acide N,N,N-triméthyl benzylammoniocarboxylique.

Les sels peuvent notamment être issus d'un acide organique, tels que des citrates, des lactates ou des tartrates, ou issus d'un acide minéral, tels que des halogénures, et notamment des chlorures, des bromures, des iodures ou des sulfates.

De préférence, ces sels sont des chlorures.

De préférence, on emploie le chlorure de l'acide N,N,N-triméthylammonioacétique, aussi appelé chlorure de bétaïnyle.

De manière connue, les deux procédés de préparation ci-dessus peuvent comporter une ou plusieurs étapes facultatives ultérieures, telles que notamment des étapes d'échanges d'anions ou de purification, par exemple par ultrafiltration.

Les composés selon l'invention peuvent être présents dans les compositions cosmétiques ou dermatologiques selon l'invention à une concentration comprise entre, de préférence, 0,01 et 20% en poids par rapport au poids total de la composition, notamment 0,05 et 15% en poids, et préférentiellement 0,1 et 10% en poids.

Les compositions cosmétiques ou dermatologiques selon l'invention comprennent un milieu cosmétiquement ou dermatologiquement acceptable, c'est-à-dire un milieu compatible avec une application sur les matières kératiniques telles que la peau, le cuir chevelu, les muqueuses, les ongles, les cils, les sourcils, les poils et/ou les cheveux ou toute autre zone cutanée du corps ou du visage.

Ces compositions présentent de préférence un pH pouvant aller de 1 à 13, préférentiellement de 2 à 12.

Ledit milieu cosmétiquement ou dermatologiquement acceptable comprend, de préférence, de l'eau et/ou des solvants organiques cosmétiquement et/ou dermatologiquement acceptables.

Les solvants organiques peuvent représenter 1 à 98% du poids total de la composition. Ils peuvent être choisis dans le groupe constitué par les solvants organiques hydrophiles, les solvants organiques lipophiles, les solvants organiques amphiphiles ou leurs mélanges.

Parmi les solvants organiques hydrophiles, on peut citer par exemple des mono-alcools linéaires ou ramifiés ayant de 1 à 8 atomes de carbone comme l'éthanol, le propanol, le butanol, l'isopropanol, l'isobutanol; des polyéthylèneglycols ayant de 6 à 80 moles d'oxyde d'éthylène; des polyols tels que le propylène glycol, le butylène glycol, le glycérol, le sorbitol; des mono- ou di-alkyle d'isosorbide dont les groupements alkyle ont 1 à 5 atomes de carbone comme le diméthyl isosorbide; des éthers de glycol comme le diéthylèneglycol monométhyléther ou diéthylèneglycol monoéthyléther; des éthers de propylène glycol comme le dipropylèneglycol méthyléther.

Comme solvants organiques lipophiles, on peut citer par exemple les esters gras liquides tels que l'adipate de diisopropyle, l'adipate de dioctyle, les benzoates d'alkyle.

Comme solvants organiques amphiphiles, on peut citer des polyols tels que des dérivés de polypropylène glycol (PPG) tels que les esters de polypropylène glycol et d'acide gras, de PPG et d'alcool gras comme le PPG-23 oléyl éther et le PPG-36 oléate.

Les compositions selon l'invention peuvent comprendre un ou plusieurs monosaccharides, oligosaccharides et/ou polysaccharides, hydrolysés ou non, modifiés ou non, identiques ou non, différents des composés selon l'invention. Ces composés peuvent notamment être choisis parmi ceux décrits dans "Encyclopedia of polymer science and engineering, John Wiley and Sons, Second Edition, 1988, volume 13, pp. 87-162, et volume 12, pp 658-690", dans "Polymers in Nature, par E. A. MacGREGOR et C. T. GREENWOOD, Editions John Wiley & Sons, Chapter 6, pp 240-328, 1980" et dans l'Industrial Gums - Polysaccharides and their Derivatives, Edité par Roy L. WHISTLER, Second Edition, Edition Academic Press Inc."

On peut notamment citer les glucanes, notamment les β-glucanes; les amidons modifiés ou non, tels que ceux issus de céréales comme le blé, le maïs ou le riz, de légumes comme le pois blond, de tubercules comme les pommes de terre ou le manioc; l'amylose, l'amylopectine, le glycogène, les dextranes; les celluloses et leurs dérivés tels que méthylcelluloses, hydroxyalkylcelluloses, éthylhydroxyéthylcelluloses, carboxyméthylcelluloses; les fructosanes, l'inuline, la levane, les mannanes, les xylanes, les lignines, les arabanes, les galactanes, les galacturonanes; la chitine, les chitosanes et leurs dérivés; les glucoronoxylanes, les arabinoxylanes, les xyloglucanes; les glucomannanes; les acides pectiques et les pectines; l'acide alginique et les alginates; les arabinogalactanes, les carraghénanes, les agars, les glycosaminoglucanes, les gommes arabiques, les gommes Tragacanthe, les gommes Ghatti, les gommes Karaya, les gommes de caroube, les gommes de xanthane, les cyclodextrines et leurs mélanges.

Les compositions selon l'invention peuvent également comprendre un ou plusieurs acides aminés, oligopeptides, peptides et/ou protéines hydrolysées ou non, modifiées ou non. Comme acides aminés, on peut citer la cystéine, la lysine, l'alanine, la N-phénylalanine, l'arginine, la glycine, la leucine et leurs mélanges.

Parmi les oligopeptides, peptides, protéines hydrolysées ou non, modifiées ou non, on peut notamment citer les hydrolysats de protéines de laine ou de soie, modifiées ou non, les protéines végétales telles que les protéines de blé.

Les compositions selon l'invention peuvent comprendre un ou plusieurs acides et alcools gras ramifiés ou non. Parmi les acides gras convenant dans la présente invention, on peut notamment citer les acides carboxyliques en C₈-C₃₀, tels que l'acide palmitique, l'acide oléique, l'acide linoléique, l'acide myristique, l'acide stéarique, l'acide laurique, et leurs mélanges. Les alcools gras utilisables dans la présente invention, comprennent notamment les alcools en C₈-C₃₀ comme, par exemple, les alcools palmitylique, oléique, linoléylique, myristylique, stéarylique, laurylique et leurs mélanges.

Les compositions selon l'invention peuvent comprendre une ou plusieurs cires d'origine animale, végétale ou minérale.

Une cire, au sens de la présente invention, est un composé lipophile, solide à température ambiante (environ 25°C), à changement d'état solide/liquide réversible, ayant une température de fusion supérieure à environ 40°C pouvant aller jusqu'à 200°C, et présentant à l'état solide une organisation cristalline anisotrope.

D'une manière générale, la taille des cristaux de la cire est telle que les cristaux diffractent et/ou diffusent la lumière, conférant à la composition qui les comprend un aspect trouble plus ou moins opaque. En portant la cire à sa température de fusion, il est possible de la rendre miscible aux huiles et de former un mélange homogène microscopiquement, mais en ramenant la température du mélange à la température ambiante, on obtient une recristallisation de la cire dans les huiles du mélange, détectable microscopiquement et macroscopiquement (opalescence).

Parmi les cires utilisables dans la présente invention, on peut citer les cires d'origine animale telles que la cire d'abeille, le spermaceti, la cire de lanoline et les dérivés de lanoline; les cires d'origine végétale telles que la cire de Carnauba, de Candellila, d'Ouricury, du Japon, le beurre de cacao ou les cires de fibres de liège ou de canne à sucre; les cires d'origine minérale telles que les cires de paraffine, de vaseline, de lignite ou les cires microcristallines ou les ozokérites, et leurs mélanges.

Les compositions selon l'invention peuvent comprendre un ou plusieurs céramides et/ou pseudo-céramides. On peut citer en particulier les céramides des classes I, II, III et V selon la classification de DOWNING, et leurs mélanges, et plus particulièrement la N-oléyldéshydrosphingosine.

Les compositions selon l'invention peuvent comprendre un ou plusieurs acides organiques hydroxylés choisis parmi ceux bien connus et utilisés dans la technique. On peut notamment citer l'acide citrique, l'acide lactique, l'acide tartrique, l'acide malique et leurs mélanges.

Les compositions selon l'invention peuvent comprendre un ou plusieurs filtres solaires actifs dans l'UV-A et/ou l'UV-B bien connus de l'homme de métier. On peut notamment citer les dérivés du dibenzoylméthane tels que le 4-méthyldibenzoylméthane, le 4-isopropyldibenzoylméthane, le 4-*tert-*butyldibenzoylméthane, le 2,4-diméthyldibenzoylméthane, le 4-*tert*-butyl-4'-diisopropyldibenzoyl-méthane, l'acide *para*-amino-benzoïque et ses esters tels que le *para*-diméthylaminobenzoate de 2-éthylhexyle et le *para*-aminobenzoate d'éthyle N-propoxylé, les salicylates tels que le salicylate de triéthanolamine, les esters d'acide cinnamique tels que le 4-méthoxycinnamate de 2-éthylhexyle, le diisopropylcinnamate de méthyle, l'anthranilate de menthyle, les dérivés de benzotriazole, les dérivés de triazine, les dérivés de β,β'-diphénylacrylate tels que le 2-cyano-3,3-diphénylacrylate de 2-éthylhexyle et le 2-cyano-3,3-diphénylacrylate d'éthyle, l'acide 2-phénylbenzimidazole-5-sulfonique et ses sels, les dérivés de benzophénone, les dérivés du benzylidènecamphre, les filtres siliconés, et leurs mélanges.

Les compositions selon l'invention peuvent comprendre un ou plusieurs antioxydants et agents anti-radicaux libres. On peut citer, par exemple, l'acide ascorbique, des composés ascorbylés tels que le dipalmitate d'ascorbyle, la t-butylhydroquinone, les polyphénols tels que le phloroglucinol, le sulfite de sodium, l'acide érythorbique, les flavonoïdes et leurs mélanges.

Les compositions selon l'invention peuvent comprendre un ou plusieurs chélatants choisis notamment parmi l'EDTA (acide éthylènediaminetétraacétique) et ses sels, tels que l'EDTA disodique et l'EDTA dipotassique, les composés phosphatés tels que le métaphosphate de sodium, l'hexamétaphosphate de sodium, le pyrophosphate tétrapotassique, les acides phosphoniques et leurs sels tels que les sels de l'acide éthylènediaminetétraméthylènephosphonique, et leurs mélanges.

Les compositions selon l'invention peuvent comprendre un ou plusieurs agents antifongiques ou antibactériens choisis, par exemple, parmi :
- le chlorure de benzéthonium, le chlorure de benzalkonium, la chlorhexidine, la chloramine T, la chloramine B, la 1,3-dibromo-5,5-diméthylhydantoïne, la 1,3-dichloro- 5,5-diméthylhydantoïne, la 3-bromo 1-chloro- 5,5-diméthylhydantoïne, le N-chlorosuccinimide ;
- les dérivés de 1-hydroxy-2-pyridone tels que, par exemple, le 1-hydroxy-4-méthyl-2-pyridone, le 1-hydroxy-6-méthyl-2-pyridone et le 1-hydroxy-4,6-diméthyl-2-pyridone ;
- les trihalogénocarbamides ;
- le triclosan ;
- les composés azolés tels que le climbazole, le kétoconazole, le clotrinazole, l'econazole, l'isoconazole et le miconazole b ;
- les polymères antifongiques tels que l'amphotéricine B ou la nystatine ;
- les sulfures de sélénium ;
- le soufre sous ses différentes formes, le sulfure de cadmium, l'allantoïne, les goudrons de houille ou de bois et leurs dérivés en particulier, l'huile de cade, l'acide undécylénique, l'acide fumarique, les allylamines telles que la terbinafine;
- ou un mélange de ces agents.

On peut également les utiliser sous forme de leurs sels d'addition d'acides physiologiquement acceptables, notamment sous forme de sels d'acides sulfurique, nitrique, thiocyanique, chlorhydrique, bromhydrique, iodhydrique, phosphorique, acétique, benzoïque, glycolique, acéturique, succinique, nicotinique, tartrique, maléique, palmitique, méthanesulfonique, propanoïque, 2-oxopropanoïque, propanedioïque, 2-hydroxy-1,4-butanedioïque, 3-phényl-2-propènoïque, hydroxybenzèneacétique, éthanesulfonique, 2-hydroxyéthanesulfonique, 4-méthylbenzènesulfonique, 4-amino-2-hydroxybenzoïque, 2-phénoxybenzoïque, 2-acétyloxybenzoïque, picrique, lactique, citrique, malique, oxalique et d'aminoacides.

Les agents antifongiques ou antibactériens mentionnés ci-dessus peuvent aussi le cas échéant être utilisés sous forme de leurs sels d'addition de bases organiques ou inorganiques physiologiquement acceptables. Des exemples de bases organiques sont notamment les alcanolamines de faibles poids moléculaires telles que l'éthanolamine, la diéthanolamine, la N-éthyléthanolamine; la triéthanolamine, le diéthylaminoéthanol, le 2-amino-2-méthylpropanedione; les bases non-volatiles telles que l'éthylènediamine, l'hexaméthylènediamine, la cyclohexylamine, la benzylamine, la N-méthylpipérazine; les hydroxydes d'ammonium quaternaires, par exemple, le triméthylbenzyl hydroxyde; la guanidine et ses dérivés, et particulièrement ses dérivés alkylés. Des exemples de bases inorganiques sont notamment les sels de métaux alcalins, comme le sodium ou potassium; les sels d'ammonium, les sels de métaux alcalino-terreux, comme le magnésium, calcium; les sels de métaux di, tri ou tétravalents cationiques, comme le zinc, l'aluminium et le zirconium. Les alcanolamines, l'éthylènediamine et les bases inorganiques telles que les sels de métaux alcalins sont préférées.

Les compositions selon l'invention peuvent comprendre un ou plusieurs agents régulateurs de séborrhée tels que le succinylchitosane et la poly-β-alanine, et leurs mélanges

Les compositions selon l'invention peuvent comprendre un ou plusieurs agents apaisants tels que l'azulène et l'acide glycyrrhétinique, et leurs mélanges.

Les compositions selon l'invention peuvent comprendre un ou plusieurs polymères cationiques. Par "polymère cationique", on entend tout polymère contenant des groupements cationiques et/ou des groupements ionisables en groupements cationiques. Les polymères cationiques utilisables conformément à la présente invention peuvent être choisis parmi tous ceux déjà connus en soi comme améliorant les propriétés cosmétiques des cheveux traités par des compositions détergentes, à savoir notamment ceux décrits dans la demande de brevet EP-A-0 337 354 et dans les demandes de brevets français FR-A- 2 270 846, 2 383 660, 2 598 611, 2 470 596 et 2 519 863.

Les polymères cationiques préférés sont choisis parmi ceux qui contiennent des motifs comportant des groupements amine primaires, secondaires, tertiaires et/ou quaternaires pouvant soit faire partie de la chaîne principale polymère, soit être portés par un substituant latéral directement relié à celle-ci.

Les polymères cationiques utilisés ont généralement de préférence une masse molaire moyenne en nombre ou en masse comprise entre 500 et 5.10⁶ environ, et de préférence comprise entre 10³ et 3.10⁶ environ.

Parmi les polymères cationiques, on peut citer plus particulièrement les polymères du type polyamine, polyaminoamide et polyammonium quaternaire, et notamment ceux décrits dans les brevets français FR2505348 et FR2542997. En particulier, on peut citer :
(1) les homopolymères ou copolymères dérivés d'esters ou d'amides de l'acide acrylique ou méthacrylique ;
(2) les dérivés d'éthers de cellulose comportant des groupements ammonium quaternaire décrits dans le brevet français 1 492 597 ;
(3) les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les hydroxyalkylcelluloses, comme les hydroxyméthyl, hydroxyéthyl ou hydropropylcelluloses greffées notamment avec un sel de méthacryloyléthyltriméthylammonium, de méthacrylamidopropyltriméthylammonium ou de diméthyldiallylammonium ;
(4) les polysaccharides cationiques décrits plus particulièrement dans les brevets US 3 589 578 et 4 031 307 tels que les gommes de guar contenant des groupements cationiques trialkylammonium ;
(5) les polymères constitués de motifs pipérazinyle et de groupes divalents alkylène ou hydroxyalkylène à chaînes linéaires ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2 162 025 et 2 280 361 ;
(6) les polyaminoamides solubles dans l'eau, tels que ceux notamment décrits dans les brevets français 2 252 840 et 2 368 508 ;
(7) les dérivés de polyaminoamides, par exemple, les polymères acide adipique/diakylaminohydroxyalkyldialkylène-triamine dans lesquels le groupe alkyle comporte de 1 à 4 atomes de carbone et désigne de préférence un groupe méthyle, éthyle, propyle, et le groupe alkylène comporte de 1 à 4 atomes de carbone, et désigne de préférence le groupe éthylène. De tels polymères sont notamment décrits dans le brevet français 1 583 363.
(8) les polymères obtenus par réaction d'une polyalkylène-polyamine comportant deux groupements amine primaire et au moins un groupement amine secondaire, avec un acide dicarboxylique choisi parmi l'acide diglycolique et les acides dicarboxyliques aliphatiques saturés ayant de 3 à 8 atomes de carbone. Le rapport molaire entre la polyalkylène-polylamine et l'acide dicarboxylique étant compris entre 0,8 : 1 et 1,4 : 1 ; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3 227 615 et 2 961 347 ;
(9) les cyclopolymères d'alkyldiallylamine ou de dialkyldiallylammonium tels que l'homopolymère de chlorure de diméthyldiallyl-ammonium et les copolymères de chlorure de diallyldiméthylammonium et d'acrylamide ;
(10) les polymères de diammonium quaternaire présentant une masse moléculaire moyenne en nombre généralement comprise entre 1000 et 100000, tels que, ceux décrits, par exemple, dans les brevets français 2 320 330, 2 270 846, 2 316 271, 2 336 434 et 2 413 907 et les brevets US 2 273 780, 2 375 853, 2 388 614, 2 454 547, 3 206.462, 2 261 002, 2 271 378, 3.874.870, 4.001.432, 3 929 990, 3 966 904, 4 005 193, 4 025 617, 4 025 627, 4 025 653, 4 026 945 et 4 027 020 ;
(11) les polymères de polyammonium quaternaire tels que ceux notamment décrits dans la demande de brevet EP-A-122 324 ;
(12) les polymères quaternaires de vinylpyrrolidone et de vinylimidazole tels que, par exemple, les produits commercialisés sous les dénominations Luviquat^{®} FC 905, FC 550 et FC 370 par la société BASF ;
(13) les polyamines comme le Polyquart^{®} H vendu par COGNIS, référencées sous le nom de « POLYETHYLENEGLYCOL (15) TALLOW POLYAMINE » dans le dictionnaire CTFA ;
(14) les polymères réticulés de sels de méthacryloyloxyalkyl(C₁-C₄) trialkyl(C₁-C₄)ammonium tels que ceux commercialisés sous le nom de SALCARE^{®} SC 92, SALCARE^{®} SC 95 et SALCARE^{®} SC 96 par la Société ALLIED COLLOIDS ; et leurs mélanges.

D'autres polymères cationiques utilisables dans le cadre de l'invention sont des protéines cationiques ou des hydrolysats de protéines cationiques, des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi les polymères cationiques mentionnés ci-dessus, on utilise de préférence les dérivés d'éther de cellulose quaternaires, les cyclopolymères cationiques, les polymères quaternaires de vinylpyrrolidone et de vinylimidazole et leurs mélanges.

Les compositions selon l'invention peuvent comprendre un ou plusieurs polymères amphotères. Les polymères amphotères utilisables conformément à l'invention peuvent être choisis parmi les polymères comportant des motifs B et C répartis statistiquement dans la chaîne polymère où B désigne un motif dérivant d'un monomère comportant au moins un atome d'azote basique et C désigne un motif dérivant d'un monomère acide comportant un ou plusieurs groupements carboxyliques ou sulfoniques ou bien B et C peuvent désigner des groupements dérivant de monomères zwittérioniques de carboxybétaïnes ou de sulfobétaïnes ; B et C peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un groupe hydrocarboné, ou bien B et C font partie d'une chaîne d'un polymère à motif éthylène-αβ-dicarboxylique dont l'un des groupements carboxyliques a été amené à réagir avec une polyamine comportant un ou plusieurs groupements amine primaire ou secondaire.

Les polymères amphotères plus particulièrement préférés peuvent être choisis parmi les polymères suivants :
1) les polymères résultant de la copolymérisation d'un monomère dérivé d'un composé vinylique portant un groupement carboxylique tel que plus particulièrement l'acide acrylique, l'acide méthacrylique, l'acide maléique, l'acide alpha-chloracrylique, et d'un monomère basique dérivé d'un composé vinylique substitué contenant au moins un atome basique tel que plus particulièrement les dialkylaminoalkylméthacrylate et acrylate, les dialkylaminoalkyl-méthacrylamide et acrylamide. De tels composés sont décrits dans le brevet américain n° 3 836 537.
   On peut également citer le copolymère acrylate de sodium/chlorure d'acrylamidopropyltriméthylammonium vendu sous la dénomination POLYQUART^{®} KE 3033 par la Société COGNIS.
   Le composé vinylique peut être également un sel de dialkyldiallylammonium tel que le chlorure de diéthyldiallylammonium.
   Les copolymères d'acide acrylique et de ce dernier monomère sont proposés sous les appellations MERQUAT^{®} 280, MERQUAT^{®} 295 et MERQUAT^{®} PLUS 3330 par la société CALGON.
(2) les polymères comportant des motifs dérivant :
   a) d'au moins un monomère choisi parmi les acrylamides ou les méthacrylamides substitués sur l'azote par un groupe alkyle,
   b) d'au moins un comonomère acide contenant un ou plusieurs groupements carboxyliques réactifs, et
   c) d'au moins un comonomère basique tel que des esters à substituants amine primaire, secondaire, tertiaire et quaternaire des acides acrylique et méthacrylique, et le produit de quaternisation du méthacrylate de diméthylaminoéthyle avec le sulfate de diméthyle ou de diéthyle.

   Les acrylamides ou méthacrylamides N-substitués plus particulièrement préférés selon l'invention sont les groupements dont les groupes alkyle contiennent de 2 à 12 atomes de carbone et plus particulièrement le N-éthylacrylamide, le N-tertiobutylacrylamide, le N-tertiooctylacrylamide, le N-octylacrylamide, le N-décylacrylamide, le N-dodécylacrylamide ainsi que les méthacrylamides correspondants.
   Les comonomères acides sont choisis plus particulièrement parmi les acides acrylique, méthacrylique, crotonique, itaconique, maléique, fumarique ainsi que les monoesters d'alkyle ayant 1 à 4 atomes de carbone des acides ou des anhydrides maléique ou fumarique. Les comonomères basiques préférés sont des méthacrylates d'aminoéthyle, de butylaminoéthyle, de N,N'-diméthylaminoéthyle, de N-tertiobutylaminoéthyle. On utilise particulièrement les copolymères dont la dénomination CTFA (4ème Ed, 1991) est Octylacrylamide/ acrylates/butylaminoethylmethacrylate copolymer tels que les produits vendus sous la dénomination-AMPHOMER^{®} ou LOVOCRYL^{®} 47 par la société NATIONAL STARCH.
(3) les polyaminoamides réticulés et alkylés partiellement ou totalement dérivant de polyaminoamides de formule générale : 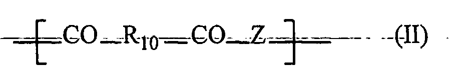 dans laquelle R₁₀ représente un groupe divalent dérivé d'un acide dicarboxylique saturé, d'un acide aliphatique mono ou dicarboxylique à double liaison éthylénique, d'un ester d'un alcanol ayant 1 à 6 atomes de carbone de ces acides ou d'un groupe dérivant de l'addition de l'un quelconque desdits acides avec une amine bis primaire ou bis dérivé secondaire, et Z désigne un groupe d'une polyalkylène-polyamine bis-primaire, mono ou bis-secondaire et représente de préférence :
   a) dans les proportions de 60 à 100 % en moles, le groupe 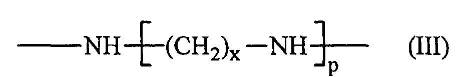 où x=2 et p=2 ou 3, ou bien x=3 et p=2
      ce groupe dérivant de la diéthylènetriamine, de la triéthylènetétraamine ou de la dipropylènetriamine;
   b) dans les proportions de 0 à 40 % en moles, le groupe (III) ci-dessus, dans lequel x=2 et p=1 et qui dérive de l'éthylènediamine, ou le groupe dérivant de la pipérazine 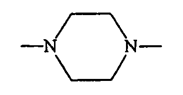
   c) dans les proportions de 0 à 20 % en moles, le groupe -NH-(CH₂)₆-NH- dérivant de l'hexaméthylènediamine, ces polyaminoamines étant réticulées par addition d'un agent réticulant bifonctionnel choisi parmi les épihalohydrines, les diépoxydes, les dianhydrides, les dérivés bis insaturés, au moyen de 0,025 à 0,35 mole d'agent réticulant par groupement amine du polyaminoamide et alcoylés par action d'acide acrylique, d'acide chloracétique ou d'une alcane-sultone ou de leurs sels.

   Les acides carboxyliques saturés sont choisis de préférence parmi les acides ayant 6 à 10 atomes de carbone tels que les acides adipique, 2,2,4-triméthyladipique et 2,4,4-triméthyladipique, téréphtalique, les acides à double liaison éthylénique comme, par exemple, les acides acrylique, méthacrylique, itaconique. Les alcane-sultones utilisées dans l'alcoylation sont de préférence la propane- ou la butane-sultone, les sels des agents d'alcoylation sont de préférence les sels de sodium ou de potassium.
(4) les polymères comportant des motifs zwittérioniques de formule : 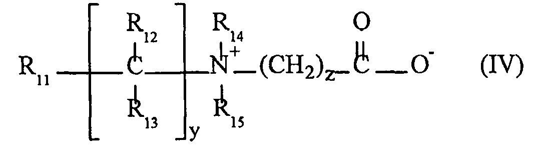 dans laquelle R₁₁ désigne un groupement insaturé polymérisable tel qu'un groupement acrylate, méthacrylate, acrylamide ou méthacrylamide, y et z représentent chacun un nombre entier de 1 à 3, R₁₂ et R₁₃ représentent un atome d'hydrogène, un groupement méthyle, éthyle ou propyle, R₁₄ et R₁₅ représentent un atome d'hydrogène ou un groupe alkyle de telle façon que la somme des atomes de carbone dans R₁₄ et R₁₅ ne dépasse pas 10.
   Les polymères comprenant de tels motifs peuvent également comporter des motifs dérivés de monomères non zwittérioniques tels que l'acrylate ou le méthacrylate de diméthyl- ou diéthylaminoéthyle, ou des acrylates ou méthacrylates d'alkyle, des acrylamides ou méthacrylamides, ou l'acétate de vinyle.
   A titre d'exemple, on peut citer le copolymère de méthacrylate de méthyle et de diméthyl-carboxyméthylammonioéthylméthacrylate de méthyle tel que le produit vendu sous la dénomination DIAFORMER^{®} Z301 par la société SANDOZ.
(5) les polymères dérivés du chitosane comportant des motifs monomères répondant aux formules suivantes: 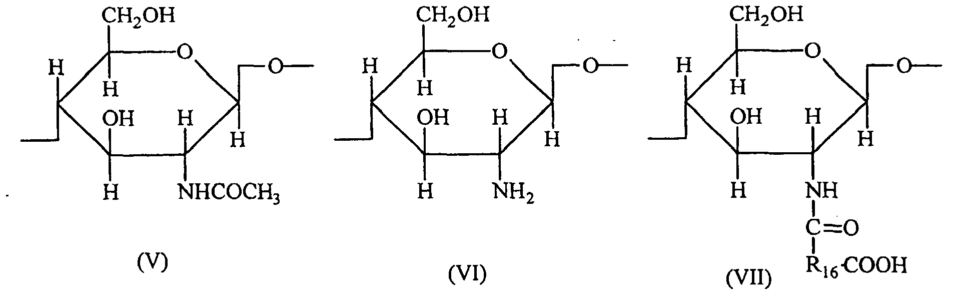 le motif (V) étant présent dans des proportions comprises entre 0 et 30 %, le motif (VI) dans des proportions comprises entre 5 et 50 % et le motif (VII) dans des proportions comprises entre 30 et 90 %, étant entendu que dans ce motif F, R₁₆ représente un groupe de formule: 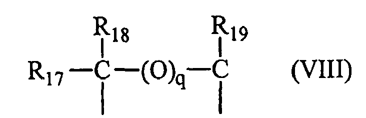 dans laquelle si q=0, R₁₇, R₁₈ et R₁₉, identiques ou différents, représentent chacun un atome d'hydrogène, un reste méthyle, hydroxyle, acétoxy ou amino, un reste monoalkylamine ou un reste dialkylamine éventuellement interrompus par un ou plusieurs atomes d'azote et/ou éventuellement substitués par un ou plusieurs groupes amine, hydroxyle, carboxyle, alkylthio, sulfonique, un reste alkylthio dont le groupe alkyle porte un reste amino, l'un au moins des groupes R₁₇, R₁₈ et R₁₉ étant dans ce cas un atome d'hydrogène ;
   ou si q=1, R₁₇, R₁₈ et R₁₉ représentent chacun un atome d'hydrogène, ainsi que les sels formés par ces composés avec des bases ou des acides.
(6) Les polymères dérivés de la N-carboxyalkylation du chitosane comme le N-carboxyméthyl-chitosane ou le N-carboxybutylchitosane vendu sous la dénomination EVALSAN^{®} par la société JAN DEKKER.
(7) Les polymères répondant à la formule générale (IX) décrits par exemple, dans le brevet français 1 400 366: 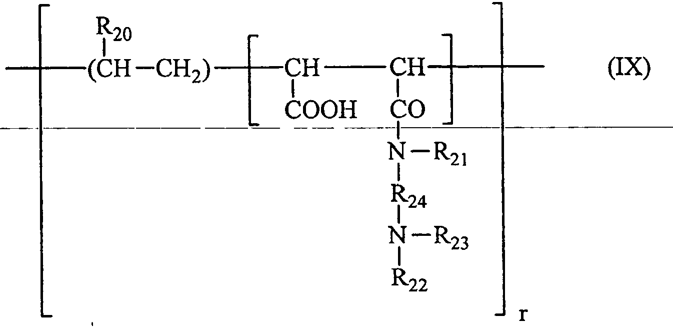 dans laquelle R₂₀ représente un atome d'hydrogène, un groupe CH₃O, CH₃CH₂O, phényle, R₂₁ désigne l'hydrogène ou un groupe alkyle inférieur tel que méthyle, éthyle, R₂₂ désigne l'hydrogène ou un groupe alkyle inférieur tel que méthyle, éthyle, R₂₃ désigne un groupe alkyle inférieur tel que méthyle, éthyle ou un groupe répondant à la formule: -R₂₄-N(R₂₂)₂, R₂₄ représentant un groupement -CH₂-CH₂-, -CH₂-CH₂-CH₂-, -CH₂-CH(CH₃)-, R₂₂ ayant les significations mentionnées ci-dessus,
   ainsi que les homologues supérieurs de ces groupes et contenant jusqu'à 6 atomes de carbone.
(8) Des polymères amphotères du type -D-X-D-X- choisis parmi:
   a) les polymères obtenus par action de l'acide chloracétique ou le chloracétate de sodium sur les composés comportant au moins un motif de formule:

      -D-X-D-X-D- (X)

      où D désigne un groupe 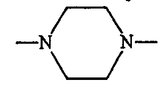 et X désigne le symbole E ou E', E ou E', identiques ou différents, désignent un groupe alkylène à chaîne droite ou ramifiée comportant 1-7 atomes de carbone dans la chaîne principale non substituée ou substituée par des groupements hydroxyle, et pouvant comporter en outre des atomes d'oxygène, d'azote, de soufre, 1 à 3 cycles aromatiques et/ou hétérocycliques; les atomes d'oxygène, d'azote et de soufre étant présents sous forme de groupements éther, thioéther, sulfoxyde, sulfone, sulfonium, alkylamine, alcénylamine, des groupements hydroxyle, benzylamine, oxyde d'amine, ammonium quaternaire, amide, imide, alcool, ester et/ou uréthane.
   b) Les polymères de formule : -D-X-D-X- (XI)
      où D désigne un groupe 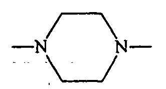 et X désigne le symbole E ou E' et au moins une fois E'; E ayant la signification indiquée ci-dessus et E' est un groupe bivalent alkylène à chaîne droite ou ramifiée ayant 1-7 atomes de carbone dans la chaîne principale, substitué ou non par un ou plusieurs groupes hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.
(9) Les copolymères alkyl(C₁-C₅)vinyléther/anhydride maléique modifié partiellement par semiamidation avec une N,N-dialkylaminoalkylamine telle que la N,N-diméthylaminopropyl-amine ou par semiestérification avec une N,N-dialcanolamine Ces copolymères peuvent également comporter d'autres comonomères vinyliques tels que le vinylcaprolactame.
   Les polymères amphotères particulièrement préférés selon l'invention sont ceux de la famille (1).

Les compositions selon l'invention peuvent comprendre un ou plusieurs polymères anioniques, solubles ou dispersés.

Les polymères anioniques de préférence utilisés dans la présente invention sont des polymères comportant des groupements carboxyliques, sulfoniques ou phosphoriques, et peuvent présenter une masse moléculaire en poids ou en masse comprise entre 500 et 5 000 000.

Les groupements carboxyliques sont apportés par des monomères monoacides ou diacides carboxyliques insaturés tels que ceux répondant à la formule : 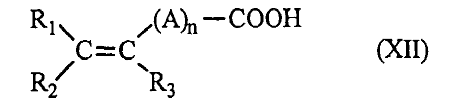 dans laquelle :
- n est un nombre entier de 0 à 10,
- A désigne un groupement méthylène, éventuellement relié à l'atome de carbone du groupement insaturé ou au groupement méthylène voisin lorsque n est supérieur à 1, par l'intermédiaire d'un hétéroatome tel que oxygène ou soufre,
- R₁ désigne un atome d'hydrogène, un groupement phényle ou benzyle,
- R₂ désigne un atome d'hydrogène, un groupement alkyle ayant 1-6 atomes de carbone, ou un groupement carboxyle,
- R₃ désigne un atome d'hydrogène, un groupement alkyle ayant 1-6 atomes de carbone, un groupement -CH₂-COOH, phényle ou benzyle.

Dans la formule (XII) ci-dessus, un groupement alkyle inférieur comporte de préférence de 1 à 4 atomes de carbone et désigne en particulier, les groupements méthyle ou éthyle.

Les polymères anioniques à groupements carboxyliques préférés selon l'invention sont :
A) les homo- ou copolymères d'acide acrylique ou méthacrylique ou leurs sels, et en particulier les produits vendus sous les dénominations VERSICOL^{®} E ou K par la société ALLIED COLLOID, ULTRAHOLD^{®} par la société BASF Les copolymères d'acide acrylique et d'acrylamide vendus sous la forme de leur sel de sodium sous les dénominations RETEN^{®} 421, 423 ou 425 par la Société HERCULES, les sels de sodium des acides polyhydroxycarboxyliques.
B) Les copolymères des acides acrylique ou méthacrylique avec un monomère monoéthylénique tel que l'éthylène, le styrène, les esters vinyliques, les esters d'acide acrylique ou méthacrylique, éventuellement greffés sur un polyalkylèneglycol tel que le polyéthylèneglycol, et éventuellement réticulés. De tels polymères sont décrits en particulier dans le brevet français 1 222 944 et la demande allemande n° 2 330 956, les copolymères de ce type comportant dans leur chaîne un motif acrylamide éventuellement N-alkylé et/ou hydroxyalkylé tels que décrits notamment dans les demandes de brevets luxembourgeois 75370 et 75371 ou proposés sous la dénomination QUADRAMER^{®} par la Société AMERICAN CYANAMID. On peut également citer les copolymères d'acide acrylique et de méthacrylate d'alkyle en C₁-C₄ et le copolymère d'acide méthacrylique et d'acrylate d'éthyle vendu sous la dénomination LUVIMER^{®} MAEX par la société BASF.
C) les copolymères dérivés d'acide crotonique tels que ceux comportant dans leur chaîne des motifs acétate ou propionate de vinyle, et éventuellement d'autres monomères tels que les esters allyliques ou méthallyliques, éthers vinyliques ou esters vinyliques d'un acide carboxylique saturé, linéaire ou ramifié, à longue chaîne hydrocarbonée, tels que ceux comportant au moins 5 atomes de carbone, ces polymères pouvant éventuellement être greffés et réticulés, ou encore un ester vinylique, allylique ou méthallylique d'un acide carboxylique α ou β cyclique. De tels polymères sont décrits entre autres dans les brevets français numéros 1 222 944, 1 580 545, 2 265 782, 2 265 781, 1 564 110 et 2 439 798. Des produits commerciaux entrant dans cette classe sont les résines 28-29-30, 26-13-14 et 28-13-10 vendues par la société NATIONAL STARCH.
D) Les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters ; ces polymères peuvent être estérifiés. De tels polymères sont décrits en particulier dans les brevets US 2 047 398, 2 723 248, 2 102 113, le brevet GB 839 805, et notamment ceux vendus sous les dénominations GANTREZ^{®} AN ou ES par la société ISP.
   Des polymères entrant également dans cette classe sont les copolymères d'anhydrides maléique, citraconique, itaconique et d'un ester allylique ou méthallylique comportant éventuellement un groupement acrylamide, méthacrylamide, une α-oléfine, des esters acryliques ou méthacryliques, des acides acryliques ou méthacryliques ou la vinylpyrrolidone dans leur chaîne, les fonctions anhydrides sont monoestérifiées ou monoamidifiées. Ces polymères sont par exemple décrits dans les brevets français 2 350 384 et 2 357 241 de la demanderesse.
E) Les polyacrylamides comportant des groupements carboxylates.

Les polymères comprenant les groupements sulfoniques sont des polymères comportant des motifs vinylsulfonique, styrène-sulfonique, naphtalène-sulfonique
ou acrylamido-alkylsulfonique.

Ces polymères peuvent être notamment choisis parmi :
- les sels de l'acide polyvinylsulfonique ayant une masse molaire comprise entre environ 1 000 et 100 000 ainsi que les copolymères avec un comonomère insaturé tel que les acides acrylique ou méthacrylique, et leurs esters, ainsi que l'acrylamide ou ses dérivés, les éthers vinyliques et la vinylprrolidone;
- les sels de l'acide polystyrènesulfonique, les sels de sodium, ayant une masse moléculaire d'environ 500 000 et d'environ 100 000 vendus respectivement sous les dénominations Flexan^{®} 500 et Flexan^{®} 130 par National Starch. Ces composés sont décrits dans le brevet FR 2198719 ;
- les sels d'acides polyacrylamide-sulfoniques tels que ceux mentionnés dans le brevet US 4 128 631, et plus particulièrement l'acide polyacrylamidoéthylpropanesulfonique vendu sous la dénomination COSMEDIA POLYMER^{®} HSP 1180 par COGNIS.

Selon l'invention, les polymères anioniques sont de préférence choisis parmi les copolymères d'acide acrylique tels que le terpolymère acide acrylique/acrylate d'éthyle/N-tertiobutylacrylamide vendu sous la dénomination ULTRAHOLD STRONG^{®} par la société BASF, les copolymères dérivés d'acide crotonique tels que les terpolymères acétate de vinyle/tertio-butylbenzoate de vinyle/acide crotonique et les terpolymères acide crotonique/acétate de vinyle/néododécanoate de vinyle vendus sous la dénomination Résine 28-29-30 par la société NATIONAL STARCH, les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters tels que le copolymère méthylvinyléther/anhydride maléïque monoestérifié vendu sous la dénomination GANTREZ^{®} ES 425 par la société ISP, les copolymères d'acide méthacrylique et de méthacrylate de méthyle vendus sous la dénomination EUDRAGIT^{®} L par la société ROHM PHARMA, le copolymère d'acide méthacrylique et d'acrylate d'éthyle vendu sous la dénomination LUVIMER^{®} MAEX par la société BASF, le copolymère acétate de vinyle/acide crotonique vendu sous la dénomination LUVISET^{®} CA 66 par la société BASF et le terpolymère acétate de vinyle/acide crotonique/polyéthylèneglycol sous la dénomination ARISTOFLEX^{®} A par la société BASF.

Les polymères anioniques les plus particulièrement préférés sont choisis parmi le copolymère méthylvinyléther/anhydride maléïque monoestérifié vendu sous la dénomination GANTREZ^{®} ES 425 par la société ISP, les copolymères d'acide méthacrylique et de méthacrylate de méthyle vendus sous la dénomination EUDRAGIT^{®} L par la société ROHM PHARMA, le copolymère d'acide méthacrylique et d'acrylate d'éthyle vendu sous la dénomination LUVIMER^{®} MAEX par la société BASF, le terpolymère de vinylpyrrolidone/acide acrylique/méthacrylate de lauryle vendu sous la dénomination ACRYLIDONE^{®} LM par la société ISP.

Selon l'invention, on peut également utiliser les polymères anioniques sous forme de latex ou de pseudolatex, c'est-à-dire sous forme d'une dispersion aqueuse de particules de polymères insolubles.

Les compositions selon l'invention peuvent comprendre un ou plusieurs polymères non-ioniques solubles ou dispersés. On peut notamment citer :
- les homopolymères de vinylpyrrolidone ;
- les copolymères de vinylpyrrolidone et d'acétate de vinyle ;
- les polyalkyloxazolines telles que les polyéthyloxazolines proposées par la société DOW CHEMICAL sous les dénominations PEOX^{®} 50 000, PEOX^{®} 200 000 et PEOX^{®} 500 000 ;
- les homopolymères d'acétate de vinyle tels que le produit proposé sous le nom de APPRETAN^{®} EM par la société HOECHST ou le produit proposé sous le nom de RHODOPAS^{®} A 012 par la société RHODIA ;
- les copolymères d'acétate de vinyle et d'ester acrylique tels que le produit proposé sous le nom de RHODOPAS^{®} AD 310 de RHODIA;
- les copolymères d'acétate de vinyle et d'éthylène comme le produit proposé sous le nom de APPRETAN^{®} TV par la société HOECHST;
- les copolymères d'acétate de vinyle et d'ester maléique, par exemple de maléate de dibutyle tels que le produit proposé sous le nom de APPRETAN^{®} MB EXTRA par la société HOECHST ;
- les copolymères de polyéthylène et d'anhydride maléique ;
- les homopolymères d'acrylates d'alkyle et les homopolymères de méthacrylates d'alkyle tels que le produit proposé sous la dénomination MICROPEARL^{®} RQ 750 par la société MATSUMOTO ou le produit proposé sous la dénomination LUHYDRAN^{®} A 848 S par la société BASF ;
- les copolymères d'esters acryliques tels que, par exemple, les copolymères d'acrylates d'alkyle et de méthacrylates d'alkyle comme les produits proposés par la société ROHM & HAAS sous les dénominations PRIMAL^{®} AC-261 K et EUDRAGIT^{®} NE 30 D, par la société BASF sous les dénominations ACRONAL^{®} 601, LUHYDRAN^{®} LR 8833 ou 8845, par la société HOECHST sous les dénominations APPRETAN^{®} N 9213 ou N921 2 ;
- les copolymères d'acrylonitrile et d'un monomère non ionique choisi, par exemple, parmi le butadiène et les (méth)acrylates d'alkyle; on peut citer les produits proposés sous les dénominations NIPOL^{®} LX 531 8 par la société NIPPON ZEON ou ceux proposés sous la dénomination CJ 0601 8 par la société ROHM & HAAS;
- les polyuréthanes tels que les produits proposés sous les dénominations ACRYSOL^{®} RM 1020 ou ACRYSOL^{®} RM 2020 par la société ROHM & HAAS, les produits URAFLEX^{®} XP 401 UZ, URAFLEX^{®} XP 402 UZ par la société DSM RESINS ;
- les copolymères d'acétate d'alkyle et d'uréthane tels que le produit 8538-33 par la société NATIONAL STARCH ;
- les polyamides tels que le produit ESTAPOR^{®} LO 11 proposé par la société RHONE POULENC;
- les gommes de guar non ioniques chimiquement modifiées ou non modifiées.

Parmi les gommes de guar non ioniques non modifiées, on peut citer les produits vendus sous la dénomination VIDOGUM® GH 175 par la société UNIPECTINE et sous la dénomination JAGUAR® C par la société MEYHALL. Parmi les gommes de guar non-ioniques modifiées, on peut citer les gommes de guar modifiées par des groupements hydroxyalkyle en C1-C6 tels que des groupements hydroxyméthyle, hydroxyéthyle, hydroxypropyle et hydroxybutyle. Ces gommes de guar sont bien connues de l'état de la technique et peuvent, par exemple, être préparées en faisant réagir des oxydes d'alcènes correspondants, tels que, par exemple, des oxydes de propylène, avec la gomme de guar de façon à obtenir une gomme de guar modifiée par des groupements hydroxypropyle. De telles gommes de guar non-ioniques éventuellement modifiées par des groupements hydroxyalkyle sont, par exemple, vendues sous les dénominations commerciales JAGUAR^{®} HP8, JAGUAR^{®} HP60 et JAGUAR^{®} HP120, JAGUAR^{®} DC 293 et JAGUAR^{®} HP 105 par la société RHODIA, ou sous la dénomination GALACTASOL^{®} 4H4FD2 par la société AQUALON.

Les groupes alkyle des polymères non ioniques comportent de préférence de 1 à 6 atomes de carbone.

Les compositions selon l'invention peuvent comprendre une ou plusieurs silicones.

Les silicones utilisables peuvent être solubles ou insolubles dans la composition, et elles peuvent être en particulier des polyorganosiloxanes insolubles dans la composition de l'invention; elles peuvent se présenter sous forme d'huiles, de cires, de résines ou de gommes. Les organopolysiloxanes sont définis plus en détail dans l'ouvrage de Walter NOLL "Chemistry and Technology of Silicones" (1968) Academic Press. Elles peuvent être volatiles ou non volatiles.

Lorsqu'elles sont volatiles, les silicones sont plus particulièrement choisies parmi celles possédant un point d'ébullition compris entre 60°C et 260°C, et plus particulièrement encore parmi :
(i) les silicones cycliques comportant de 3 à 7 atomes de silicium et, de préférence, 4 à 5. Il s'agit, par exemple, de l'octaméthylcyclotétrasiloxane commercialisé notamment sous le nom de "VOLATILE SILICONE 7207" par UNION CARBIDE ou "SILBIONE 70045 V 2" par RHODIA, le décaméthylcyclopentasiloxane commercialisé sous le nom de "VOLATILE SILICONE 7158" par UNION CARBIDE, "SILBIONE 70045 V 5" par RHODIA, ainsi que leurs mélanges.
   On peut également citer les cyclocopolymères du type diméthylsiloxane/méthylalkylsiloxane, tel que la "SILICONE VOLATILE FZ 3109" commercialisée par la société UNION CARBIDE, de structure chimique : 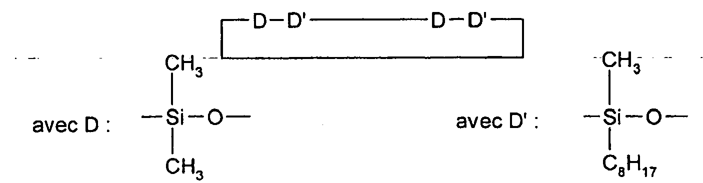
   On peut également citer les mélanges de silicones cycliques avec des composés organiques-dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'-(hexa-2,2,2',2',3,3'-triméthylsilyloxy) bisnéopentane ;
(ii) les silicones volatiles linéaires ayant 2 à 9 atomes de silicium et possédant une viscosité inférieure ou égale à 5.10⁻⁶m²/s à 25°C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and toiletries, Vol. 91, Jan. 76, p. 27-32 - TODD & BYERS "Volatile Silicone fluids for cosmetics".

Parmi les silicones non volatiles, on peut notamment citer les polyalkylsiloxanes, les polyarylsiloxanes, les polyalkylarylsiloxanes, les gommes et les résines de silicones, les polyorganosiloxanes modifiés par des groupements organofonctionnels ainsi que leurs mélanges.

Les silicones organomodifiées utilisables conformément à l'invention sont des silicones telles que définies précédemment et comportant dans leur structure un ou plusieurs groupements organofonctionnels fixés par l'intermédiaire d'un groupe hydrocarboné. Parmi les silicones organomodifiées, on peut citer les polyorganosiloxanes comportant :
- des groupements polyéthylèneoxy et/ou polypropylèneoxy comportant éventuellement des groupements alkyle en C₆-C₂₄ tels que les produits dénommés diméthicone-copolyol commercialisé par la société DOW CORNING sous la dénomination DC 1248 ou les huiles SILWET^{®} L 722, L 7500, L 77, L 711 de la société UNION CARBIDE et l'alkyl(C₁₂)-méthicone-copolyol commercialisée par la société DOW CORNING sous la dénomination Q2 5200 ;
- des groupements aminés substitués ou non, comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en C₁-C₄ ;
- des groupements thiols, comme les produits commercialisés sous les dénominations "GP 72 A" et "GP 71" de GENESEE ;
- des groupements alcoxylés, comme le produit commercialisé sous la dénomination "SILICONE COPOLYMER F-755" par SWS SILICONES et ABIL WAX^{®} 2428, 2434 et 2440 par la société GOLDSCHMIDT ;
- des groupements hydroxylés, comme les polyorganosiloxanes à fonction hydroxyalkyle décrits dans la demande de brevet français FR-A-85 16334 .
- des groupements acyloxyalkyle tels que, par exemple, les polyorganosiloxanes décrits dans le brevet US-A-4957732.
- des groupements anioniques du type acide carboxylique comme, par exemple, dans les produits décrits dans le brevet EP 186 507 de la société CHISSO CORPORATION, ou du type alkyl-carboxylique comme ceux présents dans le produit X-22-3701E de la société SHIN-ETSU ; 2-hydroxyalkylsulfonate ; 2-hydroxyalkylthiosulfate tels que les produits commercialisés par la société GOLDSCHMIDT sous les dénominations "ABIL^{®} S201" et "ABIL^{®} S255";
- des groupements hydroxyacylamino, comme les polyorgano-siloxanes décrits dans la demande EP 342 834. On peut citer, par exemple, le produit Q2-8413 de la société DOW CORNING.

On peut également utiliser des silicones greffées avec squelette hydrocarboné à greffons silicones ou à squelette siliconé à greffons hydrocarbonés tel que le produit commercialisé par la société 3M sous l'appellation VS80.

Les compositions selon l'invention peuvent comprendre une ou plusieurs huiles minérales, végétales ou animales. On peut notamment citer comme huiles d'origine végétale, l'huile d'amande douce, l'huile d'avocat, l'huile de ricin, l'huile d'olive, l'huile de jojoba, l'huile de tournesol, l'huile de germes de blé, l'huile de sésame, l'huile d'arachide, l'huile de pépins de raisin, l'huile de soja, l'huile de colza, l'huile de carthame, l'huile de coprah, l'huile de maïs, l'huile de noisette, le beurre de karité, l'huile de palme, l'huile de noyau d'abricot, l'huile de calophyllum; comme huile d'origine animale, le perhydrosqualène; comme huiles d'origine minérale, l'huile de paraffine et l'huile de vaseline; et leurs mélanges.

Les compositions selon l'invention peuvent comprendre un ou plusieurs polyisobutènes et poly(α-oléfines), choisis parmi ceux bien connus dans la technique.

Les compositions conformes à l'invention comprennent éventuellement un ou plusieurs tensioactifs anioniques, amphotères, non ioniques et/ou cationiques.

Ainsi, à titre d'exemple de tensioactifs anioniques utilisables, seuls ou en mélanges, dans le cadre de la présente invention, on peut citer notamment les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfinesulfonates, paraffinesulfonates ; les alkylsulfosuccinates, les alkyléthersulfosuccinates, les alkylamidesulfosuccinates; les alkylsulfosuccinamates ; les alkylsulfoacétates ; les alkylétherphosphates ; les acylsarcosinates ; les acyliséthionates, les acylglutamates et les N-acyltaurates, le radical alkyle ou acyle de tous ces différents composés comportant de préférence de 12 à 20 atomes de carbone, et le radical aryle désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyllactylates dont le radical acyle comporte 8 à 20 atomes de carbone. On peut également utiliser des tensioactifs faiblement anioniques, comme les acides d'alkyl D galactoside uroniques et leurs sels ainsi que les acides alkyl (C₆-C₂₄) éther carboxyliques polyoxyalkylénés, les acides alkyl(C₆-C₂₄)aryl éther carboxyliques polyoxyalkylénés ,les acides alkyl(C₆-C₂₄) amido éther carboxyliques polyoxyalkylénés et leurs sels, en particulier ceux comportant de 2 à 50 groupements oxyde d'éthylène, et leurs mélanges.

Parmi les tensioactifs anioniques, on préfère utiliser les sels d'alkylsulfates et d'alkyléthersulfates et leurs mélanges.

Parmi les tensioactifs non ioniques susceptibles d'être employés, on peut citer les alcools, les alpha-diols, les alkylphénols ou les acides gras polyéthoxylés, polypropoxylés ou polyglycérolés, ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitan oxyéthylénés ayant de 2 à 30 moles d'oxyde d'éthylène ; les esters d'acides gras du saccharose, les esters d'acides gras du polyéthylèneglycol, les alkylpolyglycosides, les dérivés de N-alkyl glucamine, les oxydes d'amines tels que les oxydes d'alkyl (C10-C14) amines ou les oxydes de N-acylaminopropylmorpholine.

Parmi les tensioactifs amphotères susceptibles d'être employés, on peut citer des dérivés d'amines secondaires ou tertiaires aliphatiques, dans lesquels le radical aliphatique est une chaîne linéaire ou ramifiée comportant 8 à 22 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) bétaïnes telles que les cocoamidopropylbétaînes ou les alkyl (C₈-C₂₀) amidoalkyl (C₁-C₆)sulfobétaïnes.

Parmi les dérivés d'amines, on peut citer les produits commercialisés sous les dénomination MIRANOL®, tels que décrits dans les brevets US-2 528 378 et US-2 781 354 et de structure de formule :

R₂ -CONHCH₂CH₂ -N(R₃)(R₄)(CH₂COO-)

dans laquelle R₂ désigne un radical alkyle dérivé d'un acide R₂-COOH présent dans l'huile de coprah hydrolysée, un radical heptyle, nonyle ou undécyle; R₃ désigne un groupement bêta-hydroxyéthyle et R₄ un groupement carboxyméthyle ; et

R'₂-CONHCH₂CH₂-N(B)(C)

dans laquelle B représente -CH₂CH₂OX' avec X' désigne le groupement -CH₂CH₂-COOH ou un atome d'hydrogène; C représente -(CH₂)_{z} -Y' avec z = 1 ou 2 et Y' désigne -COOH ou le radical -CH₂- CHOH - SO₃H; et R'₂ désigne un radical alkyle d'un acide R₉-COOH présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid.

A titre d'exemple on peut citer le cocoamphodiacetate commercialisé sous la dénomination commerciale MIRANOL® C2M concentré par la société RHODIA CHIMIE.

Parmi les tensioactifs cationiques susceptibles d'être employés, on peut citer les sels d'amines grasses primaires, secondaires ou tertiaires, éventuellement polyoxyalkylénées ; les sels d'ammonium quaternaire ; les dérivés d'imidazoline ; ou les oxydes d'amines à caractère cationique.

Les sels d'ammonium quaternaires sont par exemple choisis parmi:
- les sels d'ammonium quaternaires de formule générale (IV) suivante : 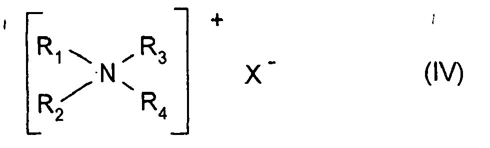 dans laquelle les radicaux R₁ à R₄, qui peuvent être identiques ou différents, représentent un radical aliphatique, linéaire ou ramifié, comportant de 1 à 30 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, polyoxyalkylène(C₂-C₆), alkylamide, alkyl(C₁₂-C₂₂)amido alkyl(C₂-C₆), alkyl(C₁₂-C₂₂)acétate, hydroxyalkyle, comportant environ de 1 à 30 atomes de carbone; X est un anion choisi dans le groupe des halogénures, phosphates, acétates, lactates, alkyl(C₂-C₆)sulfates, alkyl ou alkylarylsulfonates,
- les sels d'ammonium quaternaire de l'imidazolinium, comme par exemple celui de formule (V) suivante : 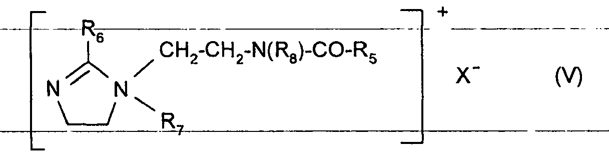 dans laquelle R₅ représente un radical alcényle ou alkyle comportant de 8 à 30 atomes de carbone par exemple dérivés des acides gras du suif, R₆ représente un atome d'hydrogène, un radical alkyle en C₁-C₄ ou un radical alcényle ou alkyle comportant de 8 à 30 atomes de carbone, R₇ représente un radical alkyle en C₁-C₄ , R₈ représente un atome d'hydrogène, un radical alkyle en C₁-C₄, X est un anion choisi dans le groupe des halogénures, phosphates, acétates, lactates, alkylsulfates, alkyl- ou alkylarylsulfonates. De préférence, R₅ et R₆ désignent un mélange de radicaux alcényle ou alkyle comportant de 12 à 21 atomes de carbone par exemple dérivés des acides gras du suif, R₇ désigne méthyle, R₈ désigne hydrogène. Un tel produit est par exemple commercialisé sous la dénomination «REWOQUAT W 75» par la société REWO,
- les sels de diammonium quaternaire de formule (VI) : 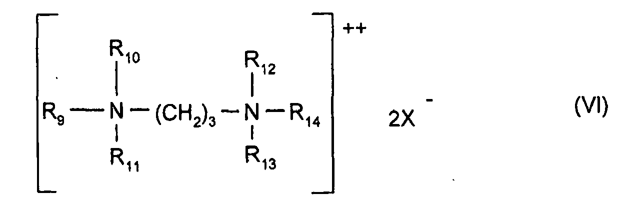 dans laquelle R₉ désigne un radical aliphatique comportant environ de 16 à 30 atomes de carbone, R₁₀, R₁₁, R₁₂, R₁₃ et R₁₄, identiques ou différents, sont choisis parmi l'hydrogène ou un radical alkyle comportant de 1 à 4 atomes de carbone, et X est un anion choisi dans le groupe des halogénures, acétates, phosphates, nitrates et méthylsulfates. De tels sels de diammonium quaternaire comprennent notamment le dichlorure de propanesuif diammonium.
- les sels d'ammonium quaternaire contenant au moins une fonction ester, et notamment ceux de formule (VII) suivante : 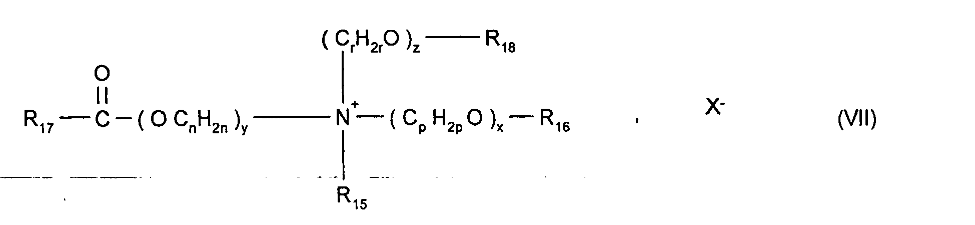
dans laquelle :
- R₁₅ est choisi parmi les radicaux alkyles, linéaires ou ramifiés, en C₁-C₆ et les radicaux hydroxyalkyles ou dihydroxyalkyles en C₁-C₆;
- R₁₆ est choisi parmi l'hydrogène; le radical R₁₉-CO- avec R₁₉ représentant un radical hydrocarboné en C₇-C₂₁, linéaire ou ramifié, saturé ou insaturé; les radicaux hydrocarbonés en C₁-C₂₂ linéaires ou ramifiés, saturés ou insaturés,
- R₁₇ représente un radical hydrocarboné en C₇-C₂₁, linéaire ou ramifié, saturé ou insaturé;
- R₁₈ est choisi parmi hydrogène ; le radical R₂₀-CO- avec R₂₀ représentant un radical hydrocarboné en C₇-C₂₁, linéaire ou ramifié, saturé ou insaturé; les radicaux hydrocarbonés en C₁-C₆ linéaires ou ramifiés, saturés ou insaturés;
- n, p et r, identiques ou différents, sont des entiers valant de 2 à 6 ;
- X- est un anion simple ou complexe, organique ou inorganique ;
- x et z, identiques ou différents, sont des entiers valant de 0 à 10 ;
- y est un entier valant de 1 à 10 ;
- la somme x+y+z vaut de 1 à 15;
sous réserve que (i) lorsque x = 0, alors R₁₆ est un radical hydrocarboné en C₁-C₂₂ linéaire ou ramifié, saturé ou insaturé; et que (ii) lorsque z = 0, alors R₁₈ est un radical hydrocarboné en C₁-C₆ linéaire ou ramifié, saturé ou insaturé.

De préférence, les radicaux alkyles R₁₅ sont linéaires, et désignent en particulier un radical méthyle, éthyle, hydroxyéthyle et dihydroxypropyle, plus particulièrement un radical méthyle ou éthyle.

Avantageusement, la somme x + y + z vaut de 1 à 10.

De préférence, lorsque R₁₆ est un radical hydrocarboné en C₁-C₂₂ linéaire ou ramifié, saturé ou insaturé, il peut avoir de 12 à 22 atomes de carbone ou de 1 à 3 atomes de carbone.

De préférence, lorsque R₁₈ est un radical hydrocarboné en C₁-C₆ linéaire ou ramifié, saturé ou insaturé, il a de préférence 1 à 3 atomes de carbone.

Avantageusement, R₁₇, R₁₉ et R₂₀, identiques ou différents, sont choisis parmi les radicaux hydrocarbonés en C₁₁-C₂₁, linéaires ou ramifiés, saturés ou insaturés, et plus particulièrement parmi les radicaux alkyle et alcényle en C₁₁-C₂₁, linéaires ou ramifiés, saturés ou insaturés.

De préférence, x et z, identiques ou différents, valent 0 ou 1.

Avantageusement, y est égal à 1.

De préférence, n, p et r, identiques ou différents, valent 2 ou 3 et encore plus particulièrement sont égaux à 2.

L'anion X est de préférence un halogénure (chlorure, bromure ou iodure) ou un alkylsulfate plus particulièrement méthylsulfate. On peut cependant utiliser le méthanesulfonate, le phosphate, le nitrate, le tosylate, un anion dérivé d'acide organique tel que l'acétate ou le lactate ou tout autre anion compatible avec l'ammonium à fonction ester. De préférence, l'anion X- est le chlorure ou le méthylsulfate.

On utilise plus particulièrement les sels d'ammonium de formule (VII) dans laquelle :
- R₁₅ désigne un radical méthyle ou éthyle,
- x et y sont égaux à 1 ;
- z est égal à 0 ou 1 ;
- n, p et r sont égaux à 2 ;
- R₁₆ est choisi parmi l'hydrogène; le radical R₁₉-CO- avec R₁₉ représentant un radical hydrocarboné en C₁₃-C₁₇, linéaire ou ramifié, saturé ou insaturé; les radicaux méthyle, éthyle ou hydrocarbonés en C₁₄-C₂₂ linéaires ou ramifiés, saturés ou insaturés;
- R₁₈ est choisi parmi l'hydrogène ou le radical R₂₀-CO- avec R₂₀ représentant un radical hydrocarboné en C₁₃-C₁₇, linéaire ou ramifié, saturé ou insaturé;
- R₁₇ est choisi parmi les radicaux hydrocarbonés en C₁₃-C₁₇, linéaires ou ramifiés, saturés ou insaturés.

Avantageusement, les radicaux hydrocarbonés sont linéaires.

On peut citer, parmi les composés de formule (VII), les sels (chlorure ou méthylsulfate notamment) de diacyloxyéthyl diméthyl ammonium, de diacyloxyéthyl hydroxyéthyl méthyl ammonium, de monoacyloxyéthyl dihydroxyéthyl méthyl ammonium, de triacyloxyéthyl méthyl ammonium, de monoacyloxyéthyl hydroxyéthyl diméthyl ammonium et leurs mélanges. Les radicaux acyle ont de préférence 14 à 18 atomes de carbone et proviennent plus particulièrement d'une huile végétale comme l'huile de palme ou de tournesol. Lorsque le composé contient plusieurs radicaux acyles, ces derniers peuvent être identiques ou différents.

Ces produits sont obtenus par exemple par estérification directe de la triéthanolamine, de la triisopropanolamine, d'alkyldiéthanolamine ou d'alkyldiisopropanolamine éventuellement oxyalkylénées sur des acides gras ou sur des mélanges d'acides gras d'origine végétale ou animale ou par transestérification de leurs esters méthyliques. Cette estérification est suivie d'une quaternisation à l'aide d'un agent alkylant tel qu'un halogénure d'alkyle (méthyle ou éthyle de préférence), un sulfate de dialkyle (méthyle ou éthyle de préférence), le méthanesulfonate de méthyle, le paratoluènesulfonate de méthyle, la chlorhydrine du glycol ou du glycérol.

De tels composés sont par exemple commercialisés sous les dénominations DEHYQUART par la société COGNIS, STEPANQUAT par la société STEPAN, NOXAMIUM par la société CECA, REWOQUAT WE 18 par la société REWO-WITCO.

On peut aussi utiliser les sels d'ammonium contenant au moins une fonction ester décrits dans les brevets US-A-4874554 et US-A-4137180.

Parmi les sels d'ammonium quaternaire de formule (IV) on préfère, d'une part, les chlorures de tétraalkylammonium comme par exemple les chlorures de dialkyldiméthylammonium ou d'alkyltriméthylammonium, dans lesquels le radical alkyle comporte environ de 12 à 22 atomes de carbone, en particulier les chlorures de béhényltriméthylammonium, de distéaryldiméthylammonium, de cétyltriméthylammonium, de benzyldiméthylstéaryl ammonium ou encore, d'autre part, le chlorure de stéaramidopropyldiméthyl (myristyl acétate) ammonium commercialisé sous la dénomination «CERAPHYL 70» par la société VAN DYK.

Dans les compositions conformes à l'invention, on peut utiliser en particulier des mélanges d'agents tensioactifs et en particulier des mélanges d'agents tensioactifs anioniques et d'agents tensioactifs amphotères ou non ioniques. Un mélange particulièrement préféré est un mélange constitué d'au moins un agent tensioactif anionique et d'au moins un agent tensioactif amphotère.

On utilise de préférence à titre d'agents tensioactifs anioniques les composés choisis parmi les alkyl(C₁₂-C₁₄) sulfates de sodium, de triéthanolamine ou d'ammonium, les alkyl (C₁₂-C₁₄)éthersulfates de sodium oxyéthylénés à 2,2 moles d'oxyde d'éthylène, le cocoyl iséthionate de sodium et l'alphaoléfine(C₁₄-C₁₆) sulfonate de sodium et leurs mélanges avec :
- soit un ou plusieurs agents tensioactifs amphotères tels que les dérivés d'amine dénommés disodiumcocoamphodipropionate ou sodiumcocoamphopropionate commercialisés notamment par la société RHODIA CHIMIE sous la dénomination commerciale "MIRANOL® C2M CONC" en solution aqueuse à 38 % de matière active ou sous la dénomination MIRANOL® C32;
- soit un ou plusieurs agents tensioactifs amphotères de type zwittérionique tel que les alkylbétaïnes en particulier la cocoylbétaïne commercialisée sous la dénomination "DEHYTON® AB 30" en solution aqueuse à 32 % de MA par la société COGNIS ou les alkylamidoalkylbétaïnes telles que la TEGOBETAINE® F50 commercialisée par la société GOLDSCHMIDT.

De façon connue, les compositions selon l'invention peuvent comprendre en outre les adjuvants habituellement utilisés dans les domaines cosmétique ou dermatologique, tels que des gélifiants et/ou épaississants associatifs ou non, de nature non ionique comme les polyuréthanes, anionique, cationique ou amphotère; des agents hydratants; des émollients; des répulsifs pour insectes; des agents amincissants; des conservateurs; des agents alcanisants ou acidifiants; des parfums; des charges minérales ou organiques; des matières colorantes; des sels minéraux ou organiques; des vitamines; des enzymes; des hormones; des antiparasitaires, des antiacnéiques, des antisudoraux, des kératolytiques, des agents antialopéciques, des agents contre les troubles de la pigmentation

Bien entendu, l'homme de l'art veillera à choisir le ou les éventuels composés complémentaires et/ou leur quantité, de manière telle que les propriétés avantageuses attachées intrinsèquement à la composition conforme à l'invention ne soient pas, ou substantiellement pas, altérées par l'addition envisagée.

D'une manière générale, les éventuels composés complémentaires peuvent être présents en une quantité qui peut varier individuellement entre 0 et 20% du poids total de la composition.

Les compositions selon l'invention peuvent se présenter sous toutes les formes appropriées pour une application topique, notamment sous forme de solutions du type lotion ou sérum ; sous forme de gels aqueux ; sous forme d'émulsions obtenues par dispersion d'une phase grasse dans une phase aqueuse (H/E) ou inversement (E/H), de consistance liquide, semi-liquide ou solide telles que des laits, des crèmes plus ou moins onctueuses, des pâtes. Ces compositions sont préparées selon les méthodes usuelles.

Les compositions selon l'invention peuvent être utilisées comme produits rincés ou non-rincés capillaires, notamment pour le lavage, le soin, le conditionnement, le maintien de la coiffure ou la mise en forme des fibres kératiniques telles que les cheveux. Elles peuvent être des produits de coiffage tels que des lotions de mise en plis, des lotions pour le brushing, des compositions de fixation et de coiffage.

Les lotions peuvent être conditionnées sous diverses formes notamment dans des vaporisateurs, des flacons pompes ou dans des récipients aérosols afin d'assurer une application de la composition sous forme vaporisée ou sous forme de mousse. De telles formes de conditionnement sont indiquées, par exemple, lorsqu'on souhaite obtenir un spray, une mousse pour la fixation ou le traitement des cheveux. Les compositions de l'invention peuvent être également des shampooings, des compositions à rincer ou non, à appliquer avant ou après un shampooing, une coloration, une décoloration, une permanente ou un défrisage.

L'invention est illustrée plus en détails dans les exemples suivants.

### Exemple 1 : Préparation d'un chlorure de bétaïnate d'amidon de blé (premier procédé)

### (composé de formule I dans laquelle Y est un reste de l'amidon, A est -CH₂-, R₁=R₂=R₃=méthyl et X est Cl)

A une solution d'amidon de blé natif (1 g, 6,17 mmol) et de N,N-diméthylglycine (0,29 g, 2,81 mmol) dans 40 ml de DMSO anhydre, on ajoute successivement 1,52 ml de N,N'-diisopropylcarbodiimide (9,88 mmol), 0,15g de 4-diméthylamino-pyridine (1,23 mmol) et 0,33 g d'hydroxybenzotriazole (2,47 mmol).

Le mélange est agité sous azote, à température ambiante pendant 12 heures, puis la réaction est stoppée par addition de 1 ml d'eau. Le mélange est versé dans 400 ml d'éthanol pour précipiter l'ester d'amidon formé.

Le précipité blanc est lavé à l'acétone et séché sous vide à température ambiante pour donner 0,85 g de poly(N,N-diméthylglycyl)ester d'amidon (rendement 74%) de degré de substitution 0,3.

A une solution de 0,75 g de poly(N,N-diméthylglycyl)ester d'amidon (4 mmol) dans 15 ml de DMSO anhydre, on ajoute 1 ml d'iodure de méthyle (16 mmol). Le mélange est agité à l'abri de la lumière et sous azote 5 heures à température ambiante. Le mélange est alors versé dans 300 ml d'éthanol pour précipiter le bétaïnate d'amidon.

Le précipité jaune est dissous dans 100 ml d'eau et on ajoute 50 ml d'une solution aqueuse de NaCI 0,05 M. La solution est ultrafiltrée au travers d'une membrane Amicon PM10 dans une cellule Amicon 8200 équipée d'un réservoir Amicon RS4 rempli de 750 ml de NaCI 0,01 M et d'eau pure. L'ultrafiltration est stoppée quand la conductivité du filtrat est inférieure à 10 µS. Le chlorure de bétaïnate d'amidon est alors récupéré sous forme de poudre blanche par lyophylisation.

On obtient 0,72 g de chlorure de bétaïnate d'amidon de blé (rendement de 89%) dont le degré de substitution déterminé par RMN du proton est de 0,3.

### Exemple 2 : Préparation d'un chlorure de bétaïnate d'amidon de blé à partir du chlorure de bétaïnyle (second procédé)

### (composé de formule I dans laquelle Y est un reste de l'amidon, A est -CH₂-, R₁=R₂=R₃=méthyl et X est Cl)

A une solution d'amidon de blé natif (1,04 g, 6,42 mmol) et de chlorure de bétaïnyle (0,394 g, 2,57 mmol) dans 40 ml de DMSO anhydre, on ajoute successivement 1,59 ml de N,N'-diisopropylcarbodiimide (10,3 mmol), 0,156 g de 4-diméthylaminopyridine (1,284 mmol) et 0,347g d'hydroxybenzotriazole (2,57 mmol).

Le mélange est agité sous azote, à température ambiante, pendant 12 heures, puis la réaction est stoppée par addition de 1 ml d'eau. Le mélange est alors versé dans 400 ml d'éthanol.

La suspension est centrifugée à 10000 tr/min pendant 12 minutes. On prélève le précipité blanc qui est lavé trois fois à l'éthanol. Le précipité est dissous dans 100 ml d'eau et la solution est filtrée sur une membrane Sartorius (taille des pores 8 à 3 µm).

Le chlorure de bétaïnate d'amidon de blé est recueilli par lyophylisation sous forme d'une poudre blanche (0,88 g, rendement : 75%).

### Exemple 3 : Préparation d'un chlorure de bétaïnate de maltodextrine

### (composé de formule I dans laquelle Y est un reste maltodextrine, A est -CH₂-, R₁=R₂=R₃=méthyl et X est Cl)

On prépare le composé recherché selon le procédé de l'exemple 1, en remplaçant l'amidon de blé par une maltodextrine de DPn = 7 (déterminée par RMN du proton) et en employant la N,N-diméthylformamide comme solvant.

On obtient un poly(N,N-diméthylglycyl)ester de maltodextrine (rendement 76%) de degré de substitution 0,3 (mesuré par RMN du proton).

Après quaternisation, la purification du dérivé quaternisé à l'iodure de méthyl dans la N,N-diméthylformamide conduit au chlorure de bétaïnate de maltodextrine recherché (rendement 85%) de degré de substitution 0,3.

### Exemple 4

On prépare un après-shampoing selon l'invention comprenant :
- Chlorure de bétaïnate de maltodextrine de l'exemple 3 0,5 g
- Chlorure de béhényl-triméthylammonium 1,5 g
- Mélange (80/20) d'alcool cétyl-stéarylique et d'alcool cétyl-stéarylique oxyéthyléné (33 motifs oxyéthylène) 4 g
- Eau qsp 100 g

Après application de l'après-shampoing sur les cheveux pendant deux minutes et rinçage, on constate que les cheveux sont doux et faciles à démêler.

### Exemple 5

On prépare une lotion de coiffage comprenant :
- Chlorure de bétaïnate de maltodextrine de l'exemple 3 0,5 g
- Terpolymère vinylcaprolactame/vinylpyrrolidone/méthacrylate de diméthylaminoéthyle, en solution à 37% dans l'éthanol
   (ADVANTAGE^{®} HC 37 de ISP) 0,5 g
- parfum, conservateur qs
- Eau qsp 100 g
   pH 6

Après application de la lotion sur cheveux et séchage, on constate que les cheveux sont doux, nerveux et se coiffent aisément.

## Revendications

1. Procédé de préparation de composés de formule (I) : 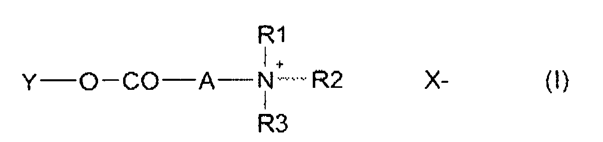 dans laquelle :
- R₁, R₂ et R₃ désignent, indépendamment l'un de l'autre, un radical hydrocarboné saturé ou insaturé, linéaire ou ramifié, en C₁-C₃₀, éventuellement interrompu par un ou plusieurs hétéroatomes choisis parmi N, O et S et/ou éventuellement substitué par un ou plusieurs radicaux choisis parmi -OH, halogène et/ou aryle en C₆-C₂₂,
- A désigne un radical hydrocarboné divalent, saturé ou insaturé, linéaire ou ramifié en C₁-C₂₂, éventuellement interrompu par un ou plusieurs hétéroatomes choisis parmi N, O et S, et/ou éventuellement substitué par un ou plusieurs radicaux hydroxyle,
- X⁻ désigne un anion issu d'un acide minéral ou organique, et
- Y désigne un reste polysaccharide,
dans lequel :
- on fait réagir un polysaccharide avec un acide N,N-dialkylaminocarboxylique de formule (II) : 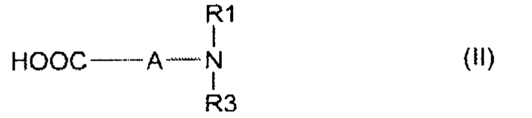 en présence d'au moins un activateur de réaction,
- puis on quaternise l'ester formé à l'aide d'un agent de quaternisation.

2. Procédé selon la revendication 1, dans lequel la réaction entre le polysaccharide et l'acide N,N-dialkylaminocarboxylique est effectuée dans un solvant polaire et aprotique.

3. Procédé selon la revendication 2, dans lequel ledit solvant polaire et aprotique est choisi parmi le diméthyl sulfoxyde (DMSO) et/ou le diméthylformamide.

4. Procédé selon l'une des revendications précédentes, dans lequel l'agent de quaternisation est un halogénure tel qu'un chlorure, un iodure ou un bromure, et notamment un halogénure d'alkyle en C1-C30, ou un sulfate, notamment un sulfate d'alkyle en C1-C30, et plus particulièrement est choisi parmi l'iodure de méthyle (Mel) ou le sulfate de méthyle (Me₂ SO₄).

5. Procédé selon l'une des revendications précédentes, dans lequel la quaternisation est effectuée à une température comprise entre 20 et 70°C, préférentiellement comprise entre 22 et 27°C.

6. Procédé selon l'une des revendications précédentes, dans lequel les acides N,N-dialkylaminocarboxyliques sont choisis parmi la N,N-diméthyl glycine (ou acide N,N-diméthyl-aminoacétique), l'acide N,N-diméthyl-β-aminopropanoïque, l'acide N,N-diméthyl-γ-aminobutanoïque, l'acide N,N-diméthyl-5-aminopentanoïque, l'acide N,N-diméthyl benzylamine carboxylique.

7. Procédé de préparation de composés de formule (I) : 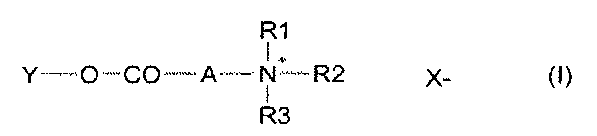 dans laquelle:
- R₁, R₂ et R₃ désignent, indépendamment l'un de l'autre, un radical hydrocarboné saturé ou insaturé, linéaire ou ramifié, en C₁-C₃₀, éventuellement interrompu par un ou plusieurs hétéroatomes choisis parmi N, O et S et/ou éventuellement substitué par un ou plusieurs radicaux choisis parmi -OH, halogène et/ou aryle en C₆-C₂₂,
- A désigne un radical hydrocarboné divalent, saturé ou insaturé, linéaire ou ramifié en C₁-C₂₂, éventuellement interrompu par un ou plusieurs hétéroatomes choisis parmi N, O et S, et/ou éventuellement substitué par un ou plusieurs radicaux hydroxyle,
- X⁻ désigne un anion issu d'un acide minéral ou organique, et
- Y désigne un reste polysaccharide,
dans lequel on fait réagir un polysaccharide avec un sel d'acide N,N,N-trialkylammonio-carboxylique de formule (III) 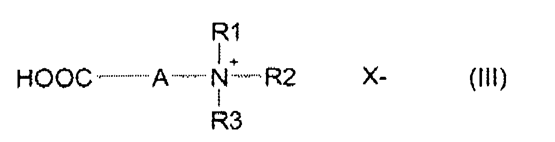 **caractérisé par le fait que :**
- la réaction est effectuée dans un solvant polaire et aprotique, et
- la réaction a lieu en présence d'au moins un activateur de réaction.

8. Procédé selon la revendication 7, dans lequel le solvant polaire et aprotique est choisi parmi le diméthyl sulfoxyde (DMSO) et/ou le diméthylformamide.

9. Procédé selon l'une des revendications 7 à 8, dans lequel les sels d'acides N,N,N-trialkylammoniocarboxyliques sont choisis parmi les sels de l'acide N,N,N-triméthyl-ammonioacétique, de l'acide N,N,N-triméthyl-β-ammoniopropanoïque, de l'acide N,N,N-triméthyl-γ-ammoniobutanoïque, de l'acide N,N,N-triméthyl-5-ammoniopentanoïque, de l'acide N,N,N-triméthyl benzylammoniocarboxylique.

10. Procédé selon l'une des revendications 7 à 9, dans lequel les sels sont choisis parmi les citrates, les lactates, les tartrates, les halogénures, et notamment les chlorures, les bromures, les iodures ou les sulfates.

11. Procédé selon l'une des revendications précédentes, dans lequel l'activateur de réaction est choisi parmi les carbodiimides tels que le N,N'-diisopropylcarbodiimide; l'hydroxybenzotriazole, le 4-diméthylamino-pyridine et leurs mélanges.

12. Composition cosmétique capillaire comprenant, dans un milieu cosmétiquement ou dermatologiquement acceptable, au moins un composé de formule (I) présent à une concentration comprise entre 0,01 et 20% en poids par rapport au poids total de la composition : 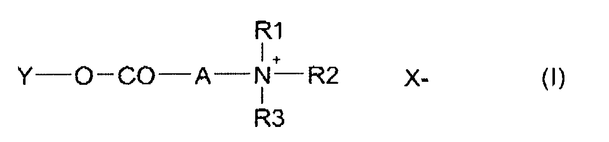 dans laquelle :
- R₁, R₂ et R₃ désignent, indépendamment l'un de l'autre, un radical hydrocarboné saturé ou insaturé, linéaire ou ramifié, en C₁-C₃₀, éventuellement interrompu par un ou plusieurs hétéroatomes choisis parmi N, O et S et/ou éventuellement substitué par un ou plusieurs radicaux choisis parmi -OH, halogène et/ou aryle en C₆-C₂₂,
- A désigne un radical hydrocarboné divalent, saturé ou insaturé, linéaire ou ramifié en C₁-C₂₂, éventuellement interrompu par un ou plusieurs hétéroatomes choisis parmi N, O et S, et/ou éventuellement substitué par un ou plusieurs radicaux hydroxyle,
- X⁻ désigne un anion issu d'un acide minéral ou organique, et
- Y désigne un reste polysaccharide,
à l'exclusion des composés dans lesquels Y représente une structure polymère de l'amidon, A représente -CH₂- et R1=R2=R3= -CH₃.

13. Composition selon la revendication 12, dans laquelle R1, R2 et R3 désignent, indépendamment l'un de l'autre, un radical hydrocarboné saturé, linéaire ou ramifié, en C₁-C₁₄, notamment en C₂-C₁₂, et préférentiellement un radical méthyle.

14. Composition selon l'une des revendications 12 à 13, dans laquelle A désigne un radical hydrocarboné divalent saturé, linéaire ou ramifié en C₁-C₄, de préférence méthylène, éthylène, propylène, butylène, et préférentiellement méthylène.

15. Composition selon l'une des revendications 12 à 14, dans laquelle les polysaccharides sont choisis parmi les amidons et notamment amidons issus de sources végétales telles que le maïs, la pomme de terre, l'avoine, le riz, le tapioca, le sorgho, l'orge ou le blé; les polysaccharides issus des gommes de graines tels que les gommes de guar, tara, caroube, psyllium, lin, okra, tamarind, coing; polysaccharides issus des gommes microbiennes telles que les gommes de curdlane, pullulane, dextrane, grifolane, schizophyllane, spirulinane, krestin, xanthane, scleroglucane, gellane, succinoglycane; les polyfructoses tels que l'inuline ou la levane; les extraits d'algues (agar, alginate, caragghénanes, fucoidane, furcellerane, laminarane); les extraits d'exudats de plantes (gomme arabique, ghatti, karaya, adraganthe); les celluloses; les chitines; les hydroxyalkylcelluloses et notamment les hydroxyéthylcelluloses, les hydroxyalkylguars tels que l'hydroxypropylguar, les carboxyalkylcelluloses, les carboxyalkylamidons, les chitosanes modifiés ou non, les maltodextrines et les cyclodextrines.

16. Composition selon l'une des revendications 12 à 15, dans laquelle les composés de formule (I) sont présents à une concentration comprise entre 0,05 et 15% en poids par rapport au poids total de la composition, préférentiellement 0,1 et 10% en poids.

17. Composition selon l'une des revendications 12 à 16, comprenant de l'eau et/ou des solvants organiques cosmétiquement et/ou dermatologiquement acceptables.

18. Composition selon la revendication 17, dans laquelle les solvants organiques représentent 1 à 98% du poids total de la composition.

19. Composition selon l'une des revendications 17 à 18, dans laquelle les solvants organiques sont choisis dans le groupe constitué par les solvants organiques hydrophiles, les solvants organiques lipophiles, les solvants organiques amphiphiles ou leurs mélanges.

20. Composition selon l'une des revendications 17 à 19, dans laquelle les solvants organiques sont choisis parmi les mono-alcools linéaires ou ramifiés ayant de 1 à 8 atomes de carbone comme l'éthanol, le propanol, le butanol, l'isopropanol, l'isobutanol; les polyéthylèneglycols ayant de 6 à 80 moles d'oxyde d'éthylène; les polyols tels que le propylène glycol, le butylène glycol, le glycérol, le sorbitol; les mono- ou di-alkyle d'isosorbide dont les groupements alkyle ont 1 à 5 atomes de carbone comme le diméthyl isosorbide; les éthers de glycol comme le diéthylèneglycol monométhyléther ou diéthylèneglycol monoéthyléther; les éthers de propylène glycol comme le dipropylèneglycol méthyléther; les esters gras liquides tels que l'adipate de diisopropyle, l'adipate de dioctyle, les benzoates d'alkyle; des polyols tels que des dérivés de polypropylène glycol (PPG) tels que les esters de polypropylène glycol et d'acide gras, de PPG et d'alcool gras comme le PPG-23 oléyl éther et le PPG-36 oléate.

21. Composition selon l'une des revendications 12 à 20, comprenant en outre au moins un composé complémentaires choisis parmi les monosaccharides, oligosaccharides et/ou polysaccharides, hydrolysés ou non, modifiés ou non; les acides aminés, oligopeptides, peptides et/ou protéines hydrolysées ou non, modifiées ou non; les acides et alcools gras ramifiés ou non; les cires d'origine animale, végétale ou minérale; les céramides et/ou pseudo-céramides; les acides organiques hydroxylés; les filtres solaires actifs dans l'UV-A et/ou l'UV-B; les antioxydants et agents anti-radicaux libres; les chélatants; les agents antifongiques ou antibactériens; les agents régulateurs de séborrhée; les agents apaisants; les polymères cationiques; les polymères amphotères; les polymères anioniques, solubles ou dispersés; les polymères non-ioniques solubles ou dispersés; les silicones; les huiles minérales, végétales ou animales; les polyisobutènes et poly(α-oléfines); les tensioactifs anioniques, amphotères, non ioniques et/ou cationiques; des gélifiants et/ou épaississants associatifs ou non, de nature non ionique comme les polyuréthanes, anionique, cationique ou amphotère; des agents hydratants; des émollients; des répulsifs pour insectes; des agents amincissants; des conservateurs; des agents alcanisants ou acidifiants; des parfums; des charges minérales ou organiques; des matières colorantes; des sels minéraux ou organiques; des vitamines; des enzymes; des hormones; des antiparasitaires, des antiacnéiques, des antisudoraux, des kératolytiques, des agents antialopéciques, des agents contre les troubles de la pigmentation.

22. Composition selon l'une des revendications 12 à 21, se présentant sous la forme de produits de coiffage tels que des lotions de mise en plis, des lotions pour le brushing, des compositions de fixation et de coiffage; de shampooings, de compositions à rincer ou non, à appliquer avant ou après un shampooing, une coloration, une décoloration, une permanente ou un défrisage.

23. Composition cosmétique selon l'une des revendications 12 à 15, se présentant sous la forme d'un produit rincé ou non rincé capillaire pour le lavage, le soin, le conditionnement, le maintien de la coiffure ou la mise en forme des cheveux.

24. Procédé de traitement cosmétique des cheveux, **caractérisé par le fait qu'**on applique sur lesdits cheveux une composition telle que définie dans l'une quelconque des revendications 12 à 23.

## Claims

1. Process for preparing compounds of formula (I): 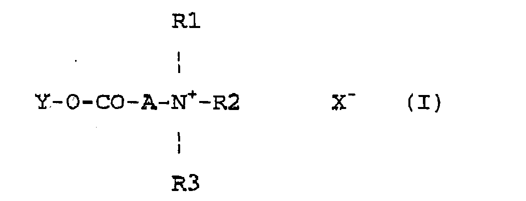 in which:
- R₁, R₂ and R₃ independently designate a linear or branched, saturated or unsaturated C₁-C₃₀ hydrocarbon radical optionally interrupted by one or more heteroatoms selected from N, O and S and/or optionally substituted with one or more radicals selected from -OH, halogen and/or C₆-C₂₂ aryl;
- A designates a linear or branched, saturated or unsaturated divalent C₁-C₂₂ hydrocarbon radical, optionally interrupted by one or more heteroatoms selected from N, O and S, and/or optionally substituted with one or more hydroxyl radicals;
- X⁻ designates an anion deriving from a mineral or organic acid; and
- Y designates a polysaccharide residue;
in which:
- a polysaccharide is reacted with an N,N-dialkylaminocarboxylic acid of formula (II): 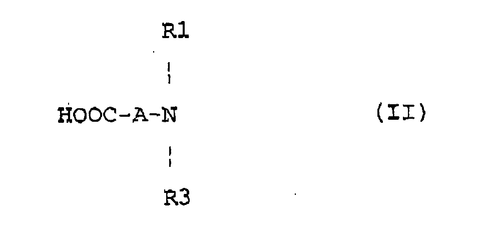 in the presence of at least one reaction activator;
- then the ester formed is quaternized using a quaternization agent.

2. Process according to Claim 1, in which the reaction between the polysaccharide and the N,N-dialkylaminocarboxylic acid is carried out in a polar aprotic solvent.

3. Process according to Claim 2, in which said polar aprotic solvent is selected from dimethylsulphoxide (DMSO) and/or dimethylformamide.

4. Process according to one of the preceding claims, in which the quaternization agent is a halide such as a chloride, an iodide or a bromide, in particular a C1-C30 alkyl halide, or a sulphate, in particular a C1-C30 alkyl sulphate, and more particularly is selected from methyl iodide (MeI) or methyl sulphate (Me₂SO₄).

5. Process according to one of the preceding claims, in which quaternization is carried out at a temperature in the range 20°C to 70°C, preferably in the range 22°C to 27°C.

6. Process according to one of the preceding claims, in which the N,N-dialkylaminocarboxylic acids are selected from N,N-dimethylglycine (or N,N-dimethylaminoacetic acid), N,N-dimethyl-β-aminopropanoic acid, N,N-dimethyl-γ-aminobutanoic acid, N,N-dimethyl-5-aminopentanoic acid and N,N-dimethylbenzylamine carboxylic acid.

7. Process for preparing compounds of formula (I): 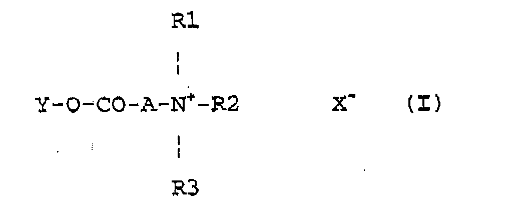 in which:
- R₁, R₂ and R₃ independently designate a linear or branched, saturated or unsaturated C₁-C₃₀ hydrocarbon radical optionally interrupted by one or more heteroatoms selected from N, O and S and/or optionally substituted with one or more radicals selected from -OH, halogen and/or C₆-C₂₂ aryl;
- A designates a linear or branched, saturated or unsaturated divalent C₁-C₂₂ hydrocarbon radical, optionally interrupted by one or more heteroatoms selected from N, O and S, and/or optionally substituted with one or more hydroxyl radicals;
- X⁻ designates an anion deriving from a mineral or organic acid; and
- Y designates a polysaccharide residue;
in which a polysaccharide is reacted with a salt of an N,N,N-trialkylammoniocarboxylic acid of formula (III): 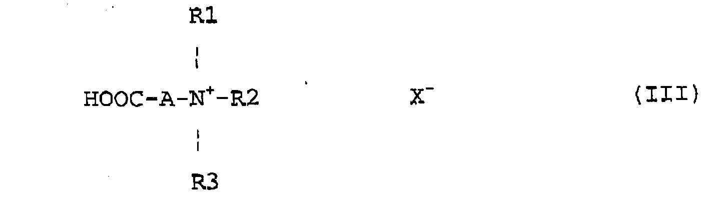 **characterized in that**:
- the reaction is carried out in a polar aprotic solvent; and
- the reaction takes place in the presence of at least one reaction activator.

8. Process according to Claim 7, in which the polar aprotic solvent is selected from dimethylsulphoxide . (DMSO) and/or dimethylformamide.

9. Process according to Claim 7 or Claim 8, in which the N,N,N-trialkylammoniocarboxylic acid salts are selected from salts of N,N,N-trimethylammonioacetic acid, N,N,N-trimethyl-β-ammoniopropanoic acid, N,N,N-trimethyl-γ-ammoniobutanoic acid, N,N,N-trimethyl-5-ammoniopentanoic acid and N,N,N-trimethylbenzylammoniocarboxylic acid.

10. Process according to one of Claims 7 to 9, in which the salts are selected from citrates, lactates, tartrates, halides, in particular chlorides, bromides or iodides, or sulphates.

11. Process according to one of the preceding claims, in which the reaction activator is selected from carbodiimides such as N,N'-diisopropylcarbodiimide; hydroxybenzotriazole, 4-dimethylaminopyridine and mixtures thereof.

12. Hair cosmetic composition comprising, in a cosmetically or dermatologically acceptable medium, at least one compound of formula (I) that is present in a concentration in the range 0.01% to 20% by weight with respect to the total composition weight: 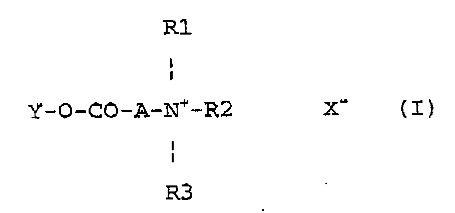 in which:
- R₁, R₂ and R₃ independently designate a linear or branched, saturated or unsaturated C₁-C₃₀ hydrocarbon radical optionally interrupted by one or more heteroatoms selected from N, O and S and/or optionally substituted with one or more radicals selected from -OH, halogen and/or C₆-C₂₂ aryl;
- A designates a linear or branched, saturated or unsaturated divalent C₁-C₂₂ hydrocarbon radical, optionally interrupted by one or more heteroatoms selected from N, O and S, and/or optionally substituted with one or more hydroxyl radicals;
- X⁻ designates an anion deriving from a mineral or organic acid; and
- Y designates a polysaccharide residue;
excluding compounds in which Y represents a polymeric starch structure, A represents -CH₂- and R1 = R2 = R3 = -CH₃.

13. Composition according to Claim 12, in which R1, R2 and R3 independently designate a linear or branched, saturated C₁-C₁₄ hydrocarbon radical, in particular C₂-C₁₂, and preferably a methyl radical.

14. Composition according to Claim 12 or Claim 13, in which A designates a linear or branched, saturated divalent C₁-C₄ hydrocarbon radical, preferably methylene, ethylene, propylene, butylene, and preferably methylene.

15. Composition according to one of Claims 12 to 14, in which the polysaccharides are selected from starches, in particular starches from vegetable origins such as maize, potato, oats, rice, tapioca, sorghum, barley or wheat; polysaccharides from seed gums such as guar, tara, carob, psyllium, linseed, okra, tamarind, quince gums; polysaccharides from microbial gums such as curdlan, pullulan, dextran, grifolan, schizophyllan, spirulinan, krestin, xanthan, scleroglucan, gellan, succinoglycan gums; polyfructoses such as inulin or levan; algae extracts (agar, alginate, carrageenans, fucoidane, furcellerane, laminarane); extracts from plant exudates (gum arabic, ghatti gum, karaya gum, adraganth gum); celluloses; chitins; hydroxyalkyl celluloses, in particular hydroxyethyl celluloses, hydroxyalkyl guars such as hydroxypropyl guar, carboxyalkyl celluloses, carboxyalkyl starches, modified or unmodified chitosans, maltodextrins and cyclodextrins.

16. Composition according to one of Claims 12 to 15, in which the compounds of formula (I) are present in a concentration that is in the range 0.05% to 15% by weight with respect to the total composition weight, preferably 0.1% to 10% by weight.

17. Composition according to one of Claims 12 to 16, comprising water and/or cosmetically and/or dermatologically acceptable organic solvents.

18. Composition according to Claim 17, in which the organic solvents represent 1% to 98% of the total composition weight.

19. Composition according to Claim 17 or Claim 18, in which the organic solvents are selected from the group formed by hydrophilic organic solvents, lipophilic organic solvents, amphiphilic organic solvents and mixtures thereof.

20. Composition according to one of Claims 17 to 19, in which the organic solvents are selected from linear or branched monoalcohols containing 1 to 8 carbon atoms, such as ethanol, propanol, butanol, isopropanol or isobutanol; polyethylene glycols containing 6 to 80 mol of ethylene oxide; polyols such as propylene glycol, butylene glycol, glycerol or sorbitol; mono- or dialkyl isosorbide with alkyl groups that contain 1 to 5 carbon atoms such as dimethyl isosorbide; glycol ethers such as diethylene glycol monomethyl ether or diethylene glycol monoethyl ether; propylene glycol ethers such as dipropylene glycol methyl ether; liquid fatty esters such as diisopropyl adipate, dioctyl adipate or alkyl benzoates; and polyols such as derivatives of polypropylene glycol (PPG), for example esters of polypropylene glycol and fatty acids, or of PPG and fatty alcohols such as PPG-23 oleyl ether and PPG-36 oleate.

21. Composition according to one of Claims 12 to 20, further comprising at least one complementary compound selected from monosaccharides, oligosaccharides and/or polysaccharides, which may or may not be hydrolysed, and which may be modified or unmodified; amino acids, oligopeptides, peptides and/or proteins, which may or may not be hydrolysed, and which may be modified or unmodified; branched or unbranched fatty alcohols and acids; waxes of animal, vegetable or mineral origin; ceramides and/or pseudo-ceramides; hydroxylated organic acids; UV-A and/or UV-B active sunscreens; antioxidants and free radical scavengers; chelating agents; antifungal or antibacterial agents; seborrhoea-regulating agents; soothing agents; cationic polymers; amphoteric polymers; or anionic polymers, which are either soluble or dispersed; soluble or dispersed nonionic polymers; silicones ; mineral, vegetable or animal oils; polyisobutenes and poly(α-olefins) ; anionic, amphoteric, nonionic and/or cationic surfactants; gelling agents and/or thickening agents, which may or may not be associative, of a nonionic nature such as polyurethanes, or anionic, cationic or amphoteric in nature; moisturizing agents; emollients; insect repellents; thinning agents; preservatives; basifying or acidifying agents; fragrances; mineral or organic fillers; colourants; mineral or organic salts; vitamins; enzymes; hormones; antiparasitic agents, anti-acne agents, antiperspirants, keratolytic agents, anti-alopecia agents, and agents countering pigmentation problems.

22. Composition according to one of Claims 12 to 21, in the form of hairstyling products such as setting lotions, blow-drying lotions, fixing and styling compositions; shampoos, rinse or no-rinse compositions for application before or after a shampooing, a coloring, a bleaching, a perming or a straightening treatment.

23. Cosmetic composition according to one of Claims 12 to 15, in the form of a rinse or no-rinse haircare product, in particular for washing, care, conditioning, maintaining the style or shaping the hair.

24. Process for cosmetic treatment of the hair, **characterized in that** a composition as defined in any one of Claims 12 to 23 is applied to said hair.

## Patentansprüche

1. Verfahren zur Herstellung von Verbindungen der Formel (I) 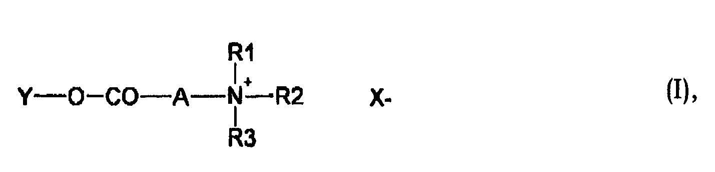 worin bedeuten:
- R₁, R₂ und R₃ unabhängig voneinander eine gesättigte oder ungesättigte, geradkettige oder verzweigte C₁₋₃₀-Kohlenwasserstoffgruppe, die gegebenenfalls durch ein oder mehrere Heteroatome unterbrochen ist, die unter N, O und S ausgewählt sind, und/oder gegebenenfalls mit einer oder mehreren Gruppen substituiert ist, die unter -OH, Halogen und/oder C₆₋₂₂-Aryl ausgewählt sind,
- A eine gesättigte oder ungesättigte, geradkettige oder verzweigte zweiwertige C₁₋₂₂-Kohlenwasserstoffgruppe, die gegebenenfalls durch ein oder mehrere Heteroatome unterbrochen ist, die unter N, O und S ausgewählt sind, und/oder gegebenenfalls mit einer oder mehreren Hydroxygruppen substituiert ist,
- X⁻ ein Anion, das von einer anorganischen oder organischen Säure stammt, und
- Y einen Polysaccharidrest,
bei dem:
- ein Polysaccharid mit einer N,N-Dialkylaminocarbonsäure der Formel (II): 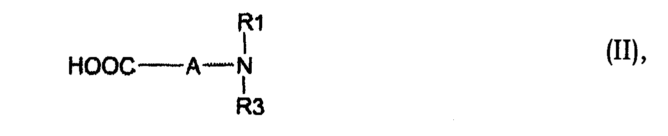
in Gegenwart mindestens eines Aktivators für die Reaktion umgesetzt wird, und
- dann der gebildete Ester mit Hilfe eines Quaternisierungsmittels quaternisiert wird.

2. Verfahren nach Anspruch 1, bei dem die Umsetzung des Polysaccharids und der N,N-Dialkylaminocarbonsäure in einem polaren und aprotischen Lösungsmittel durchgeführt wird.

3. Verfahren nach Anspruch 2, bei dem das polare und aprotische Lösungsmittel unter Dimethylsulfoxid (DMSO) und/oder Dimethylformamid ausgewählt ist.

4. Verfahren nach einem der vorhergehenden Ansprüche, bei dem das Quaternisierungsmittel ein Halogenid, wie ein Chlorid, Iodid oder Bromid, und insbesondere ein C₁₋₃₀-Alkylhalogenid oder ein Sulfat ist, insbesondere ein C₁₋₃₀-Alkylsulfat, wobei es insbesondere unter Methyliodid (MeI) oder Methylsulfat (Me₂SO₄) ausgewählt ist.

5. Verfahren nach einem der vorhergehenden Ansprüche, bei dem die Quaternisierung bei einer Temperatur von 20 bis 70 °C und vorzugsweise 22 bis 27 °C erfolgt.

6. Verfahren nach einem der vorhergehenden Ansprüche, bei dem die N,N-Dialkylaminocarbonsäuren unter N,N-Dimethylglycin (oder N,N-Dimethylaminoessigsäure), N,N-Dimethyl-β-aminopropansäure, N,N-Dimethyl-γ-aminobutansäure, N,N-Dimethyl-5-aminopentansäure und N,N-Dimethylbenzylamincarbonsäure ausgewählt sind.

7. Verfahren zur Herstellung von Verbindungen der Formel (I) 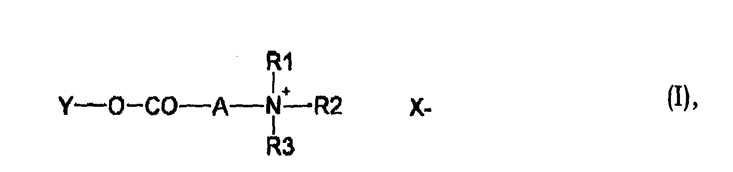 worin bedeuten:
- R₁, R₂ und R₃ unabhängig voneinander eine gesättigte oder ungesättigte, geradkettige oder verzweigte C₁₋₃₀-Kohlenwasserstoffgruppe, die gegebenenfalls durch ein oder mehrere Heteroatome unterbrochen ist, die unter N, O und S ausgewählt sind, und/oder gegebenenfalls mit einer oder mehreren Gruppen substituiert ist, die unter -OH, Halogen und/oder C₆₋₂₂-Aryl ausgewählt sind,
- A eine gesättigte oder ungesättigte, geradkettige oder verzweigte zweiwertige C₁₋₂₂-Kohlenwasserstoffgruppe, die gegebenenfalls durch ein oder mehrere Heteroatome unterbrochen ist, die unter N, O und S ausgewählt sind, und/oder gegebenenfalls mit einer oder mehreren Hydroxygruppen substituiert ist,
- X⁻ ein Anion, das von einer anorganischen oder organischen Säure stammt, und
- Y einen Polysaccharidrest,
bei dem:
- ein Polysaccharid mit einem Salz einer N,N,N-Trialkylammoniocarbonsäure der Formel (III) umgesetzt wird: 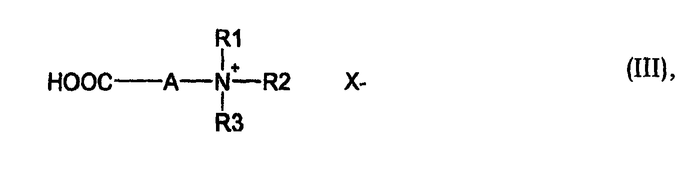
**dadurch gekennzeichnet, dass**:
- die Umsetzung in einem polaren und aprotischen Lösungsmittel erfolgt und
- die Umsetzung in Gegenwart mindestens eines Aktivators für die Reaktion durchgeführt wird.

8. Verfahren nach Anspruch 7, wobei das polare und aprotische Lösungsmittel unter Dimethylsulfoxid (DMSO) und/oder Dimethylformamid ausgewählt ist.

9. Verfahren nach einem der Ansprüche 7 bis 8, wobei die Salze der N,N,N-Trialkylammoniocarbonsäuren unter den Salzen von N,N,N-Trimethylammonioessigsäure, N,N,N-Trimethyl-β-ammoniopropansäure, N,N,N-Trimethyl-γ-ammoniobutansäure, N,N,N-Trimethyl-5-ammoniopentansäure und N,N,N-Trimethylbenzylammoniocarbonsäure ausgewählt sind.

10. Verfahren nach einem der Ansprüche 7 bis 9, wobei die Salze unter den Citraten, Lactaten, Tartraten, Halogeniden und insbesondere Chloriden, Bromiden, Iodiden oder Sulfaten ausgewählt sind.

11. Verfahren nach einem der vorhergehenden Ansprüche, wobei der Aktivator für die Reaktion unter den Carbodiimiden, wie N,N'- Diisopropylcarbodiimid; Hydroxybenzotriazol, 4-Dimethylaminopyridin und deren Gemischen ausgewählt ist.

12. Kosmetische Zusammensetzung für die Haarbehandlung, die in einem kosmetisch oder dermatologisch akzeptablen Medium mindestens eine Verbindung der Formel (I) enthält, die in einer Konzentration von 0,01 bis 20 Gew.-%, bezogen auf das Gesamtgewicht der Zusammensetzung, vorliegt: 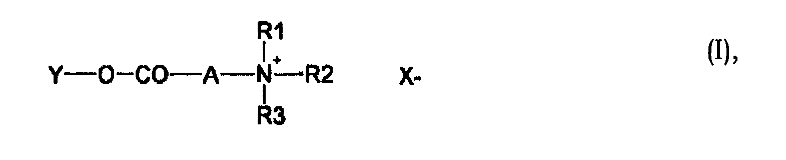 worin bedeuten:
- R₁, R₂ und R₃ unabhängig voneinander eine gesättigte oder ungesättigte, geradkettige oder verzweigte C₁₋₃₀-Kohlenwasserstoffgruppe, die gegebenenfalls durch ein oder mehrere Heteroatome unterbrochen ist, die unter N, O und S ausgewählt sind, und/oder gegebenenfalls mit einer oder mehreren Gruppen substituiert ist, die unter -OH, Halogen und/oder C₆₋₂₂-Aryl ausgewählt sind,
- A eine gesättigte oder ungesättigte, geradkettige oder verzweigte zweiwertige C₁₋₂₂-Kohlenwasserstoffgruppe, die gegebenenfalls durch ein oder mehrere Heteroatome unterbrochen ist, die unter N, O und S ausgewählt sind, und/oder gegebenenfalls mit einer oder mehreren Hydroxygruppen substituiert ist,
- X⁻ ein Anion, das von einer anorganischen oder organischen Säure stammt, und
- Y einen Polysaccharidrest,
wobei Verbindungen ausgenommen sind, bei denen Y eine polymere Stärkestruktur bedeutet, A -CH₂- ist und R₁=R₂=R₃=-CH₃.

13. Zusammensetzung nach Anspruch 12, wobei R₁, R₂ und R₃ unabhängig voneinander eine geradkettige oder verzweigte, gesättigte Kohlenwasserstoffgruppe mit 1 bis 14 Kohlenstoffatomen und insbesondere 2 bis 12 Kohlenstoffatomen und bevorzugt Methyl bedeutet.

14. Zusammensetzung nach einem der Ansprüche 12 oder 13, wobei A eine gesättigte, geradkettige oder verzweigte zweiwertige C₁₋₄-Kohlenwasserstoffgruppe ist, vorzugsweise Methylen, Ethylen, Propylen, Butylen, und bevorzugt Methylen.

15. Zusammensetzung nach einem der Ansprüche 12 bis 14, wobei die Polysaccharide ausgewählt sind unter den Stärkeverbindungen und insbesondere Stärke, die aus pflanzlichen Quellen stammt, wie Mais, Kartoffel, Hafer, Reis, Tapioka, Hirse, Gerste oder Weizen; Polysacchariden, die aus Samenkörnern stammen, wie Guargummi, Taragummi, Johannisbrotkernmehl, Psyllium, Leinen, Okra, Tamarindenkernmehl, Quitte; Polysacchariden, die mikrobieller Herkunft sind, wie Curdlan, Pullulan, Dextran, Grifolan, Schizophyllan, Spirulinan, Krestin, Xanthan, Skleroglucan, Gellan, Succinoglycan; Polyfructosen, wie Inulin oder Levan; Algenextrakten (Agar, Alginat, Carrageenanen, Fucoidan, Furcelleran, Laminaran); Extrakten von Pflanzenexsudaten (Gummi arabicum, Ghatti-Gummi, Karaya-Gummi, Tragant); Cellulosen; Chitinen; Hydroxyalkylcellulosen und besonders Hydroxyethylcellulosen, Hydroxyalkylguarverbindungen, wie Hydroxypropylguar, Carboxyalkylcellulosen, Carboxyalkylstärken, Chitosanen, die gegebenenfalls modifiziert sind, Maltodextrinen und Cyclodextrinen.

16. Zusammensetzung nach einem der Ansprüche 12 bis 15, wobei die Verbindungen der Formel (I) in einer Konzentration von 0,05 bis 15 Gew.-%, bezogen auf das Gesamtgewicht der Zusammensetzung, und bevorzugt 0,1 bis 10 Gew.-% enthalten sind.

17. Zusammensetzung nach einem der Ansprüche 12 bis 16, die Wasser und/oder organische Lösungsmittel enthalten, die kosmetisch und/oder dermatologisch akzeptabel sind.

18. Zusammensetzung nach Anspruch 17, wobei die organischen Lösungsmittel 1 bis 98 % des Gesamtgewichts der Zusammensetzung ausmachen.

19. Zusammensetzung nach einem der Ansprüche 17 bis 18, wobei die organischen Lösungsmittel unter den hydrophilen organischen Lösungsmitteln, lipophilen organischen Lösungsmitteln, amphiphilen organischen Lösungsmitteln oder deren Gemischen ausgewählt sind.

20. Zusammensetzung nach einem der Ansprüche 17 bis 19, wobei die organischen Lösungsmittel unter den geradkettigen oder verzweigten Monoalkoholen mit 1 bis 8 Kohlenstoffatomen, wie Ethanol, Propanol, Butanol, Isopropanol, Isobutanol; Polyethylenglycolen mit 6 bis 80 mol Ethylenoxid; Polyolen, wie Propylenglycol, Butylenglycol, Glycerin, Sorbit; Mono- oder Dialkylisosorbid, deren Alkylgruppen 1 bis 5 Kohlenstoffatome aufweisen, wie Dimethylisosorbid; Glycolethern, wie Diethylenglycolmonomethylether oder Diethylenglycolmonoethylether; Propylenglycolethern, wie Dipropylenglycolmethylether; flüssigen Fettsäureestern, wie Diisopropyladipat, Dioctyladipat, Alkylbenzoaten; Polyolen, wie Polypropylenglycolderivaten (PPG), wie Estern von Polypropylenglycol und Fettsäuren, PPG und Fettalkoholen, wie PPG-23-oleylester und PPG-36-oleat.

21. Zusammensetzung nach einem der Ansprüche 12 bis 20, die ferner mindestens eine ergänzende Verbindung enthält, die ausgewählt ist unter Monosacchariden, Oligosacchariden und/oder Polysacchariden, die hydrolysiert oder nichthydrolysiert, modifiziert oder nichtmodifiziert sind; Aminosäuren, Oligopeptiden, Peptiden und/oder Proteinen, die hydrolysiert oder nichthydrolysiert, modifiziert oder nichtmodifiziert sind; Fettsäuren und Fettalkoholen, die verzweigt oder unverzweigt sind; Wachsen tierischer, pflanzlicher oder mineralischer Herkunft; Ceramiden und/oder Pseudoceramiden; organischen Hydroxysäuren; im UV-A- und/oder UV-B-Bereich wirksamen Sonnenschutzfiltern; Antioxidantien und Radikalfängern für freie Radikale; Chelatbildnern; antimykotischen oder antibakteriellen Wirkstoffen; Seborrhö regulierenden Wirkstoffen; beruhigenden Stoffen; kationischen Polymeren; amphoteren Polymeren; anionischen Polymeren, die löslich oder dispergiert sind; nichtionischen Polymeren, die löslich oder dispergiert sind; Siliconen; Mineralölen, pflanzlichen Ölen oder tierischen Ölen; Polyisobutenen und Poly(a-olefinen); anionischen, amphoteren, nichtionischen und/oder kationischen grenzflächenaktiven Stoffen; Gelbildnern und/oder Verdickungsmitteln, die assoziativ oder nichtassoziativ und nichtionischer Natur sind, wie Polyurethanen, oder anionischer, kationischer oder amphoterer Natur; Hydratisierungsmitteln; Emollientien; insektenabwehrenden Wirkstoffen; schlanker machenden Wirkstoffen; Konservierungsmitteln; Alkalisierungsmitteln oder Ansäuerungsmitteln; Parfums; anorganischen oder organischen Füllstoffen; Farbmitteln; anorganischen oder organischen Salzen; Vitaminen; Enzymen; Hormonen; antiparasitären Wirkstoffen; Antiaknemitteln; schweißverhütenden Wirkstoffen; Keratolytika; Wirkstoffen gegen Alopezie, Wirkstoffen gegen Pigmentierungsstörungen.

22. Zusammensetzung nach einem der Ansprüche 12 bis 21, die in Form von Produkten für die Frisurengestaltung vorliegt, wie Lotionen für Wasserwellen, Lotionen zum Fönen, Zusammensetzungen für die Festigung und Frisurengestaltung; Haarwaschmitteln, Zusammensetzungen, die ausgespült werden oder im Haar verbleiben und vor oder nach einer Haarwäsche, Färbung, Entfärbung, dauerhaften Verformung oder Entkräuselung angewandt werden.

23. Kosmetische Zusammensetzung nach einem der Ansprüche 12 bis 15, die in Form eines Produktes für die Haarbehandlung vorliegt, das ausgespült wird oder im Haar verbleibt, für die Reinigung, die Pflege, die Konditionierung, den Halt der Frisur oder die Formgebung der Frisur.

24. Verfahren zur kosmetischen Behandlung der Haare, **dadurch gekennzeichnet, dass** eine Zusammensetzung nach einem der Ansprüche 12 bis 23 auf die Haare aufgebracht wird.
